# EUROPÄISCHE PATENTANMELDUNG

(11) **EP 4 541 382 A1**
(43) Veröffentlichungstag der Anmeldung: **23.04.2025**
(21) Anmeldenummer: 23203860.4
(22) Anmeldetag: 16.10.2023
(51) Int. Cl.: A61L 2/07, A61B 90/70, B08B 3/00, B01J 3/06, F22B 1/28

(54) **VERFAHREN ZUR DAMPFSTERILISATION IN EINEM AUTOKLAV SOWIE AUTOKLAV**

(71) Anmelder: MELAG Medizintechnik GmbH & Co. KG, 10829 Berlin (DE)
(72) Erfinder: VON DER WAYDBRINK, Eberhard, 14641 Paulinenaue (DE); KÜNKEL, Stephan, 15366 Hoppegarten (DE); LITZBA, Guido, 14513 Teltow (DE); BERTHE, André, 10825 Berlin (DE); BECKER, Tino, 10179 Berlin (DE); HERMANN, Martin, 12247 Berlin (DE); SEIBERT, Philipp, 13469 Berlin (DE)
(74) Vertreter: Maikowski & Ninnemann Patentanwälte Partnerschaft mbB

(57) **Zusammenfassung**

Die Erfindung betrifft ein Verfahren zur Dampfsterilisation in einem Autoklav. Das Verfahren umfasst die Erzeugung von gasförmigem Wasser in einem Sofortdampferzeuger, die Bereitstellung des gasförmigen Wassers in einer Kammer des Autoklavs zur Sterilisation von in der Kammer angeordnetem Sterilgut, sowie die Einstellung einer Sterilisationsbedingung in der Kammer für die Dauer einer Haltezeit. Das Verfahren zeichnet sich dadurch aus, dass sämtliches während der Haltezeit in der Kammer befindliches Wasser sterilisiert wird. Weiterer Gegenstand der Erfindung ist ein entsprechender Autoklav.

## Beschreibung

Die Erfindung betrifft ein Verfahren zur Dampfsterilisation in einem Autoklav nach dem Oberbegriff des Anspruchs 1 und einen entsprechenden Autoklaven nach dem Oberbegriff des Anspruchs 11.

Die Dampfsterilisation wird beispielsweise eingesetzt, um medizinische Geräte zu sterilisieren. Hierbei wird in einer Sterilisationskammer eines Dampfsterilisators oder Autoklaven Wasserdampf genutzt, um die Zahl der aktiven und vermehrungsfähigen Mikroorganismen zu reduzieren. Um eine ausreichende Reduzierung der entsprechenden Mikroorganismen zu erreichen, sehen die einschlägigen Normen vor, dass eine Sterilisationsbedingung für die Dauer einer Haltezeit in der Kammer gehalten wird. Beispielsweise sieht die EN 285 vor, dass als Sterilisationsbedingung eine Sterilisationstemperatur von 121°C über eine Haltezeit von 15 Minuten oder eine Sterilisationstemperatur von 134°C über eine Haltezeit von drei Minuten gehalten werden, jeweils bei Sattdampfbedingungen. Die EN 13060 ergänzt darüber hinaus als Sterilisationsbedingungen eine Sterilisationstemperaturen von 126°C mit einer Haltezeit von zehn Minuten und eine Sterilisationstemperatur von 143°C mit einer Haltezeit von einer Minute, beides ebenfalls bei Sattdampfbedingungen. Nur bei einer Einhaltung der Sterilisationsbedingungen über die Dauer der Haltezeit garantiert eine ausreichende Reduzierung von Mikroorganismen und damit eine Sterilisation. Darüber hinaus sorgt die Haltezeit für die Einhaltung des Sterilisationswertes.

Der Wasserdampf wird durch Erhitzen aus Wasser erzeugt. Zur Erzeugung des Wasserdampfes kommen vermehrt auch sogenannte Sofortdampferzeuger zum Einsatz. Ein Sofortdampferzeuger verdampft das ihn durchströmende Wasser instantan, so dass eine Wasserdampferzeugung unmittelbar dann erfolgt, wenn die Regelung in der Sterilisationskammer, im Folgenden kurz Kammer genannt, einen Dampfeinlass bzw. eine Druckerhöhung vorsieht. Im Gegensatz zu gewöhnlichen Dampferzeugern wird der Wasserdampf weder über einen längeren Zeitraum erzeugt, noch ist es notwendig, ihn in einem Vorratsbehälter vorzuhalten. Vielmehr erfolgt die Dampferzeugung bedarfsgerecht. Dies ermöglicht eine schnellere und effizientere Versorgung des Autoklavs mit Wasserdampf zu vergleichsweise geringen Kosten. Ein Sofortdampferzeuger wird bereits vor der Zufuhr von Speisewasser, d.h. flüssigem Wasser, aufgeheizt. Die Dampferzeugung wird dann durch die Zufuhr von Speisewasser ausgelöst. Hier ist die größere Wärmekapazität des Sofortdampferzeugers gegenüber einem konventionellen Dampferzeuger von Vorteil, die dafür sorgt, dass kein starker Temperaturabfall durch die Verdampfung stattfindet. Im Gegensatz dazu werden gewöhnliche Dampferzeuger mit flüssigem Wasser gespeist, die Dampferzeugung aber dann durch Einschalten der Heizung erst ausgelöst. Der Patentschrift EP 972 159 B1 ist beispielsweise ein Sofortdampferzeuger zu entnehmen. Wasser wird durch einen aufgeheizten Metallblock geleitet und in diesem verdampft. Der so erzeugte Wasserdampf wird in die Kammer eines Autoklavs geleitet, um hier Kammerdruck und -temperatur auf die für die Sterilisation benötigten Werte einzustellen. EP 681 141 A1, DE 69 622 316 und EP 1 516 632 A1 zeigen weitere Varianten von Sofortdampferzeugern.

Nachteilig ist hierbei allerdings, dass während der Haltezeit immer wieder neues Wasser im Sofortdampferzeuger verdampft und in die Kammer eingeleitet wird, um hier die notwendigen Druck- und/oder Temperatursteigerungen zu erreichen. Dieser Wasserdampf unterliegt aber selbst nicht für die Dauer der Haltezeit den Sterilisationsbedingungen. Der nach dem Beginn der Haltezeit in die Kammer aus dem Sofortdampferzeuger eingespeiste Wasserdampf ist somit selbst nicht steril und wird in der Kammer auch aufgrund der nicht ausreichenden verbleibenden Haltezeit auch nicht sterilisiert. Im Wasserdampf, insbesondere in darin enthaltenen Aerosoltropfen, befindliche Mikroorganismen werden somit nicht ausreichend inaktiviert, so dass der Wasserdampf in der Kammer angeordnetes Sterilgut mit Mikroorganismen kontaminieren kann. Am Ende der Haltezeit liegt somit kein steriles Sterilgut in der Kammer vor. Dies ist umso bedenklicher, da das Wasser vor der Verdampfung im Dampferzeuger demineralisiert werden muss. Hierbei kann es zu längeren Standzeiten des Wassers mit der entsprechenden Keimbildung und Verunreinigung kommen. Da bei Verwendung eines Sofortdampferzeugers etwaige im Wasser befindliche Keime nicht für die Dauer der Haltezeit den Sterilisationsbedingungen ausgesetzt sind, werden diese Keime folglich auch nicht zwangsweise unschädlich gemacht, sondern kontaminieren das eigentlich zu sterilisierende in der Kammer befindliche Gut. So beschreiben die Patentanmeldungen EP 1 623 724 A1 und WO 2009/039 616 A1 Verfahren und Vorrichtungen zur Reduktion der Biofilmbildung in einem Autoklav, stellen jedoch die Eignung von potentiell keimhaltigem Wasser für die Sterilisation nicht in Frage.

Es ist deshalb eine Aufgabe der vorliegenden Erfindung, ein Verfahren zur Dampfsterilisation mit einem Autoklav und einen Autoklaven zur Verfügung zu stellen, das bzw. der eine hygienisch einwandfreie, d.h. tatsächlich sterile, Dampfsterilisation trotz Sofortdampferzeugung ermöglicht.

Die Aufgabe wird gelöst durch ein Verfahren zur Dampfsterilisation in einem Autoklav mit den Merkmalen des Anspruchs 1. Das Verfahren umfasst den Schritt der Erzeugung von gasförmigem Wasser, auch Wasserdampf genannt, in einem Sofortdampferzeuger. In einem Sofortdampferzeuger erfolgt die Dampferzeugung instantan, beispielsweise bei Durchleitung von flüssigem Wasser durch eine Bohrung in einem heizbaren Block, insbesondere Metallblock. In dem Sofortdampferzeuger wird gasförmiges Wasser erzeugt. Das gasförmige Wasser wird in einer Kammer, insbesondere einer Sterilisationskammer zur Aufnahme von Sterilgut, des Autoklavs zur Sterilisation von in der Kammer angeordnetem Sterilgut bereitgestellt. Für die Dauer einer Haltezeit wird eine Sterilisationsbedingung in der Kammer eingestellt. Eine Sterilisationsbedingung umfasst beispielsweise eine Sterilisationstemperatur und/oder einen Sterilisationsdruck. Insbesondere umfasst die Sterilisationsbedingung eine Sterilisationstemperatur und einen Sterilisationsdruck, wobei der Sterilisationsdruck der sich bei der Sterilisationstemperatur gemäß Sattdampftabelle einstellende Druck ist. Die Haltezeit ist insbesondere in Abhängigkeit von der Sterilisationsbedingung gewählt. Zum Beispiel wird als Sterilisationsbedingung eine Sterilisationstemperatur von 121°C bei Sattdampfbedingungen für die Dauer einer Haltezeit in der Kammer eingestellt, wobei die Haltezeit für diese Sterilisationsbedingung 15 Minuten beträgt. Alternativ wird als Sterilisationsbedingung eine Sterilisationstemperatur von 134°C bei Sattdampfbedingungen für die Dauer einer Haltezeit in der Kammer eingestellt, wobei die Haltezeit für diese Sterilisationsbedingung lediglich drei Minuten beträgt. Auch andere, in den einschlägigen die Dampfsterilisation betreffenden Normen geregelte Sterilisationsbedingungen mit ihren entsprechenden Haltezeiten stellen Beispiele für Sterilisationsbedingungen und Haltezeiten im Sinne der Erfindung dar. Das Verfahren zeichnet sich dadurch aus, dass sämtliches während der Haltezeit in der Kammer befindliches Wasser sterilisiert wird, insbesondere so, dass am Ende der Haltezeit ausschließlich sterilisiertes Wasser in der Kammer vorliegt. Hierbei umfasst der Begriff "Wasser" sowohl Wasser in seiner gasförmigen als auch in seiner flüssigen Phase, also sowohl flüssiges Wasser als auch Wasserdampf. Sterilisation umfasst sowohl die Dampfsterilisation als auch die Sterilfiltration. Dabei bezeichnet im Folgenden "steril" in Übereinstimmung mit DIN EN 556 einen Zustand, in dem die theoretische Wahrscheinlichkeit, einen lebensfähigen Mikroorganismus zu finden, kleiner als 1 :1.000.000. Durch die Sterilisation sämtlichen Wassers, das während der Haltezeit in der Kammer bereitgestellt wird, wird ein hygienisch einwandfreies Verfahren zur Dampfsterilisation unter Verwendung eines Sofortdampferzeugers zur Verfügung gestellt. Eine Kontamination der Kammer mit nicht sterilisiertem Wasser, wie es bei der Dampfsterilisation mit Sofortdampferzeugung im Stand der Technik erfolgt, wird vermieden. Die Sterilisation des sämtlichen, während der Haltezeit in der Kammer bereitgestellten Wassers erfolgt vor und/oder während der Bereitstellung in der Kammer, d.h. vor und/oder während der Haltezeit, in flüssiger und/oder gasförmiger Phase.

Gemäß einem Aspekt des Verfahrens wird sämtliches während der Haltezeit in der Kammer befindliches Wasser vor der Bereitstellung in der Kammer sterilisiert oder nach der Bereitstellung in der Kammer sterilisiert oder vor der Bereitstellung in der Kammer teilsterilisiert und nach der Bereitstellung in der Kammer vollständig sterilisiert. Insbesondere wird sämtliches während der Haltezeit in der Kammer befindliches Wasser vor dem Beginn der Haltezeit sterilisiert oder während der Haltezeit sterilisiert oder vor der Haltezeit teilsterilisiert und während der Haltezeit vollständig sterilisiert. Dabei ist das Wasser beispielsweise vollständig sterilisiert, wenn ein Sterilisätssicherheitswert SAL (engl. sterility assurance level) von 10⁻⁶ erreicht wird, d.h. die theoretische Wahrscheinlichkeit, einen lebensfähigen Mikroorganismus zu finden, 1:1.000.000 oder weniger beträgt. Der SAL-Wert gibt die Wahrscheinlichkeit an, mit der ein einziger lebensfähiger Mikroorganismus auf einen Sterilgut nach der Sterilisation auftritt. Der SAL-Wert wird als negativer Exponent zur Basis 10 angegeben und ist ein quantitativer Wert. Ein SAL-Wert von 10⁻⁶ ist ein geringerer Wert, bezeichnet aber eine größere Sicherstellung von Sterilität als beispielsweise ein SAL-Wert von 10⁻³. Das Wasser ist in diesem Sinne teilsterilisiert, wenn ein SAL-Wert größer als 10⁻⁶ erreicht wurde. Als Mindesttemperatur für die mikrobielle Inaktivierung wird in der Dampfsterilisation eine Temperatur von mindestens 115°C vorgesehen, siehe z.B. Normenentwurf ISO/FDIS 17665. Ab dieser Temperatur erfolgt eine Einwirkung von Wasser, insbesondere von gasförmigem Wasser, im Sinne einer Sterilisation. Unter diesen Bedingungen wird Wasser bereits teilsterilisiert. Eine vollständige Sterilisation erfordert jedoch höhere Temperaturen bei entsprechenden Haltezeiten. Die letzte Alternative beschreibt somit eine Sterilisation des Wassers in mehreren, insbesondere zwei, Schritten. Dabei beziehen sich sowohl die Sterilisation als auch die Teilsterilisation und die vollständige Sterilisation auf Wasser in seiner gasförmigen und/oder in seiner flüssigen Phase. Durch die Sterilisation des Wassers vor der Bereitstellung in der Kammer wird sichergestellt, dass überhaupt nur steriles Wasser in die Kammer gelangt, diese also durch das Wasser nicht mit Mikroorganismen kontaminiert wird. Wird das Wasser nach Bereitstellung in der Kammer sterilisiert, wird sichergestellt, dass durch das Wasser etwaig in die Kammer gelangte Mikroorganismen in ausreichender Anzahl inaktiviert werden, um eine Sterilisation von in der Kammer gelagertem Gut sicherzustellen. Wird das Wasser vor der Bereitstellung in der Kammer lediglich teilsterilisiert, ist eine vollständige Sterilisation in der Kammer notwendig, um eine Sterilisation von in der Kammer gelagertem Gut sicherzustellen.

In einem Aspekt des Verfahrens erfolgt eine Einstellung der Sterilisationsbedingung während der Haltezeit ausschließlich durch Verdampfung von in der Kammer anfallendem Kondensat. Hierdurch wird sichergestellt, dass sämtliches in der Kammer während der Haltezeit bereitgestelltes Wasser für die Dauer der Haltezeit einer Sterilisationsbedingung unterliegt. Sämtliches in der Kammer während der Haltezeit bereitgestelltes Wasser wird somit in der Kammer sterilisiert. Insbesondere wird während der Haltezeit kein neues Wasser in die Kammer eingespeist, das dann nur für eine gegenüber der Haltezeit verkürzte Zeitdauer in der Kammer der Sterilisationsbedingung unterworfen und somit am Ende der Haltezeit nicht notwendigerweise steril wäre. In einem alternativen Aspekt des Verfahrens wird vor Beginn der Haltezeit Wasser in die Kammer gespeist. Eine Einstellung der Sterilisationsbedingung während der Haltezeit erfolgt ausschließlich durch Verdampfung des vor der Haltezeit in die Kammer gespeisten Wassers und durch Verdampfung von in der Kammer während der Haltezeit anfallendem Kondensat. Sämtliches in der Kammer während der Haltezeit befindliches Wasser unterliegt somit für die Dauer der Haltezeit einer Sterilisationsbedingung, wird somit sterilisiert.

Gemäß einem weiteren Aspekt des Verfahrens wird ein in der Kammer anfallendes Kondensat in der Kammer verdampft. In einer Variante, wird zusätzlich vor Beginn der Haltezeit in die Kammer eingespeistes Wasser in der Kammer verdampft. Über die Verdampfung des Kondensats, insbesondere des Kondensats und des vor Beginn der Haltezeit in die Kammer gespeisten Wassers, in der Kammer lässt sich eine Sterilisationsbedingung während der Haltezeit einstellen, ohne dass neues Wasser in die Kammer eingespeist werden müsste. Die Verdampfung erfolgt beispielsweise mittels eines in der Kammer angeordneten Heizelementes. Alternativ ist der Sofortdampferzeuger selbst in der Kammer angeordnet, so dass die Verdampfung mittels des Sofortdampferzeugers in der Kammer erfolgt. Ein zusätzliches Heizelement mit entsprechender Regelung wird hierbei nicht benötigt. Die Sofortdampferzeugung erfolgt direkt in der Kammer, so dass das gasförmige Wasser auch direkt hier bereitgestellt werden kann. In einer ersten Variante umfasst der Sofortdampferzeuger eine in der Kammer angeordnete Heizspirale, insbesondere einen Rohrheizkörper, wobei in die Kammer gespeistes Wasser die Heizspirale, insbesondere den Rohrheizkörper, direkt kontaktiert und so verdampft wird. Beispielsweise weist der Autoklav eine in der Kammer angeordnete und auf die Heizspirale, insbesondere den Rohrheizkörper, ausgerichtete Düse zum direkten Speisen bzw. Spritzen von flüssigem Wasser auf die Heizspirale, insbesondere den Rohrheizkörper, auf. Das Speisen mit flüssigem Wasser erfolgt insbesondere bei eingeschalteter Heizspirale, insbesondere eingeschaltetem Rohrheizkörper. Dies unterscheidet den Sofortdampferzeuger von einem gewöhnlichen Dampferzeuger, der sowohl keine Einspritzung von flüssigem Wasser direkt auf die Heizspirale, insbesondere Rohrheizkörper, vorsieht und ein Erhitzen der Heizspirale, insbesondere des Rohrheizkörpers, erst nach dem Speisevorgang beginnt.

Beispielsweise umfasst der Sofortdampferzeuger einen Heizkörper, insbesondere eine Heizspirale oder eine Heizpatrone, wobei die Heizspirale oder die Heizpatrone in einem Heizblock angeordnet ist. Der Heizblock ist beispielsweise im Bereich des Bodens der Kammer angeordnet. In einer Variante ist der Heizblock mittels Aluminiumdruckguss oder Aluminiumextrusion gefertigt. Beispielsweise weist der Autoklav eine in der Kammer angeordnete Düse zum Speisen, insbesondere Spritzen, von flüssigem Wasser auf den Heizkörper auf. Das Speisen des flüssigen Wassers erfolgt insbesondere bei eingeschaltetem Heizkörper. Der Heizkörper wirkt somit als Sofortdampferzeuger.

In einer weiteren Variante umfasst der Sofortdampferzeuger die gesamte Kammerwandung oder Teile der Kammerwandung, beispielsweise den Boden der Kammer und/oder die seitliche Kammerwandung. In der Kammerwandung, insbesondere im Kammerboden, ist in einem Beispiel mindestens ein Heizelement, insbesondere eine Heizpatrone, angeordnet, mittels dessen die Kammerwandung, insbesondere der Kammerboden, erhitzt werden und als Sofortdampferzeuger fungieren kann. Zusätzlich umfasst der Sofortdampferzeuger die seitliche Kammerwandung. In dieser Variante ist alternativ oder zusätzlich mindestens ein Heizelement in der seitlichen Kammerwandung angeordnet, mittels dessen die Kammerwandung erhitzt und zur Sofortdampferzeugung genutzt wird. Heizelemente in der seitlichen Kammerwandung sind in einer Variante lediglich vorgesehen, um die seitliche Kammerwandung zu erwärmen und eine Kondensatbildung zu vermindern oder zu unterbinden. Beispielsweise umfasst der Sofortdampferzeugung die gesamte Kammerwandung oder Teile der Kammerwandung, wobei die Kammer ein stranggepresster Kessel, insbesondere ein stranggepresster Aluminiumkessel, ist. Alternativ ist der stranggepresste Kessel aus einem anderen wärmeleitfähigen Material gefertigt, beispielsweise aus Kupfer. In einem weiteren Beispiel ist die Kammer in einem Druckgussverfahren gefertigt, insbesondere mittels Aluminiumdruckguss.

In einer weiteren Alternative ist der Sofortdampferzeuger an der Kammerwand, insbesondere am Kammerboden, angeordnet. Beispielsweise umfasst der Sofortdampferzeuger Induktionsheizelemente. In einer weiteren Variante umfasst der Sofortdampferzeuger außen an der Kammerwand angeordnete Heizkörper, insbesondere außen an der Kammerwand angeordnete Dickschichtelemente.

Die Kammer weist beispielsweise eine Düse auf, über die Wasser in die Kammer eingespritzt werden kann. Das Wasser kommt somit unmittelbar mit den erhitzten Teilen der Kammerwandung in Kontakt, die als Sofortdampferzeuger wirken. Auf die Kammerwandung, insbesondere den Kammerboden, gespritztes Wasser kann so instantan verdampft werden.

In einer Variante erfolgt eine Einstellung einer Sterilisationsbedingung in der Kammer für die Dauer einer Haltezeit mittels einer Regelungseinheit, wobei die Regelungseinheit mindestens einen Sensor aufweist. Der Sensor ist beispielsweise ein Temperatursensor oder ein Drucksensor. In einer Variante umfasst die Regelungseinheit mindestens einen Temperatursensor, wobei der mindestens eine Temperatursensor im Inneren der Kammer angeordnet ist oder im Kontakt mit der Kammerwandung im Inneren der Kammer oder außerhalb der Kammer angeordnet ist. In einer Variante ist der mindestens eine Temperatursensor an dem im Boden der Kammer angeordneten Heizelement angeordnet. Alternativ weist die Regelungseinheit mindestens einen, insbesondere genau einen, Drucksensor im Inneren der Kammer auf.

Alternativ wird in der Kammer anfallendes Kondensat, oder in der Kammer anfallendes Kondensat und vor Beginn der Haltezeit in die Kammer gespeistes Wasser, außerhalb der Kammer verdampft. Beispielsweise ist der Sofortdampferzeuger außerhalb der Kammer angeordnet und die die Verdampfung des Kondensats, oder des Kondensats und des vor Beginn der Haltezeit in die Kammer gespeisten Wassers, erfolgt in dem Sofortdampferzeuger außerhalb der Kammer. Sofortdampferzeuger und Kammer sind hier getrennt, bilden aber für das Kondensat oder für das Kondensat und das vor Beginn der Haltezeit in die Kammer gespeiste Wasser bzw. das durch dessen Verdampfung erzeugte gasförmige Wasser einen Kreislauf. Ein zusätzliches Heizelement wird nicht benötigt.

Gemäß einem Aspekt wird das in der Kammer anfallende Kondensat, oder das in der Kammer anfallende Kondensat und das vor Beginn der Haltezeit in die Kammer gespeiste Wasser, mittels einer Pumpe aus der Kammer in den außerhalb der Kammer angeordneten Sofortdampferzeuger gepumpt. Dabei können Kammer, Sofortdampferzeuger und Pumpe einen Kreislauf bilden, in dem das Kondensat oder das Kondensat und das vor Beginn der Haltezeit in die Kammer gespeiste Wasser bzw. das daraus erzeugte gasförmige Wasser während der Haltezeit nach Bedarf zirkuliert. Beispielsweise kann hier ein regelmäßig in Autoklaven vorgesehener Anschluss zur Abführung des Kondensats aus der Kammer zur Zuführung des Kondensats oder des Kondensats und des vor Beginn der Haltezeit in die Kammer gespeisten Wassers an den Sofortdampferzeuger genutzt werden. In einem Beispiel ist die Pumpe eine eigens für diesen Kreislauf vorgesehene Pumpe. In einem alternativen Beispiel wird eine Pumpe für die Einleitung von Speisewasser, d.h. flüssigem Wasser, aus einem Tank in den Sofortdampferzeuger und für das Pumpen des Kondensats, oder des in der Kammer anfallenden Kondensats und des vor Beginn der Haltezeit in die Kammer gespeisten Wassers, von der Kammer zum Sofortdampferzeuger genutzt. Beispielsweise ist ein Mehrwege-Ventil, beispielsweise ein 3/2-Wege-Ventil, vorgesehen, um die beiden Kreisläufe miteinander zu verbinden. Somit wird nur eine Pumpe sowohl zum Einleiten von Speisewasser in den Sofortdampferzeuger und zur Rückführung des Kondensats, oder des in der Kammer anfallenden Kondensats und des vor Beginn der Haltezeit in die Kammer gespeisten Wassers, zum Sofortdampferzeuger benötigt.

Gemäß einem Aspekt erfolgt eine Einstellung der Sterilisationsbedingung während der Haltezeit, insbesondere auch, durch ein Nachspeisen von gasförmigem Wasser in die Kammer. Gemäß diesem Aspekt wird anstelle oder zusätzlich zu dem vor Beginn der Haltezeit in der Kammer bereitgestelltem Wasser weiteres Wasser genutzt, um die Sterilisationsbedingung einzustellen.

Beispielsweise weist der Sofortdampferzeuger ein mittels eines Ventils gegenüber der Kammer abgeriegeltes Dampfreservoir auf. Im Sofortdampferzeuger wird gasförmiges Wasser auf Vorrat erzeugt und während der Haltezeit zur Einstellung der Sterilisationsbedingung in die Kammer geleitet. Somit wird während der Haltezeit zur Einstellung der Sterilisationsbedingung kein neues Speisewasser benötigt, das nicht für die Dauer der Haltezeit einer Sterilisationsbedingung unterworfen wäre. Das Dampfreservoir ist beispielsweise über ein Ventil gegenüber der Kammer abriegelbar, so dass eine Einstellung der Sterilisationsbedingung über Stellvorgänge am Ventil erfolgt. In einem Beispiel ist zur Vermeidung einer Totstrecke und einer Vermischungsgefahr mit neuem Speisewasser im Bereich eines Speisewasserzugangs des Sofortdampferzeugers in diesem Bereich ein Ventil, insbesondere ein Rückschlagventil angeordnet. Alternative Maßnahmen zur Vermeidung einer solchen Totstrecke und damit verbundenen Vermischungsgefahr sind der Fachperson bekannt und können ebenso Anwendung finden.

Gemäß einem Aspekt wird das Wasser vor der Zufuhr zum Sofortdampferzeuger sterilisiert. Somit ist sämtliches in den Sofortdampferzeuger gelangendes (flüssiges) Wasser bereits steril, so dass der im Sofortdampferzeuger hergestellte Wasserdampf keiner Sterilisation mehr bedarf.

Beispielsweise erfolgt die Sterilisation des Wassers in einem dem Sofortdampferzeuger vorgelagerten Vorlagebehälter durch Erhitzen auf eine Temperatur oberhalb einer Sterilisationstemperatur. Hierzu wird beispielsweise Speisewasser, z.B. aus einem Speisewassertank, in einen Vorlagebehälter eingespeist. Der Vorlagebehälter verfügt insbesondere über eine eigene Heizung, mittels der das eingespeiste Wasser auf eine Temperatur oberhalb einer Sterilisationstemperatur erhitzt wird. Beispielsweise wird das eingespeiste Wasser auf 150°C erhitzt. Der Vorlagebehälter ist in einer Variante so ausgelegt, dass er das für einen Sterilisationsvorgang während der Haltezeit benötigte Wasservolumen vorhalten kann. In einem Beispiel ist ein zwangsgesteuertes Ventil zwischen Vorlagebehälter und Sofortdampferzeuger vorgesehen, so dass ein durch das Erhitzen des Wassers auf eine Temperatur oberhalb einer Sterilisationstemperatur entstandener Überdruck genutzt werden kann, das vorgeheizte Wasser über das zwangsgesteuerte Ventil in den Sofortdampferzeuger zu überführen. In einem Beispiel ist zur Vermeidung einer Totstrecke und einer Vermischungsgefahr mit neuem Speisewasser im Bereich eines Speisewasserzugangs des Vorlagebehälters in diesem Bereich ein Ventil, insbesondere ein Rückschlagventil angeordnet. Alternative Maßnahmen zur Vermeidung einer solchen Totstrecke und damit verbundenen Vermischungsgefahr sind der Fachperson bekannt und können ebenso Anwendung finden.

Alternativ erfolgt die Sterilisation des Wassers in einer in oder an der Kammer angeordneten Spirale. Die Spirale ist ausgeführt, ein für einen Sterilisationsvorgang während der Haltezeit benötigtes Wasservolumen zu speichern. Beispielsweise wird neues Speisewasser vor der Einspeisung in den Sofortdampferzeuger über eine Spirale geleitet. Die Spirale ist in der Kammer oder an der Kammer angeordnet, so dass die Spirale durchströmendes Wasser für die Dauer der Haltezeit der Sterilisationsbedingung ausgesetzt ist. Somit ist sichergestellt, dass sämtliches zur Sterilisation verwendetes Wasser für die Dauer der Haltezeit der Sterilisationsbedingung ausgesetzt war und somit vor Bereitstellung in der Kammer sterilisiert wurde. Alternativ wird das Wasser in der Spirale lediglich teilsterilisiert. Beispielsweise wird das Wasser in der Spirale einer Mindesttemperatur zur mikrobiellen Inaktivierung unterworfen, z.B. einer Mindesttemperatur von 115°C. Ab dieser Mindesttemperatur wird das Wasser bereits teilsterilisiert, d.h. die Anzahl an aktiven Mikroorganismen reduziert. Eine vollständige Sterilisation, d.h. ein Erreichen eines SAL-Werts von 10⁻⁶, erfordert höhere Temperaturen als die Mindesttemperatur zur mikrobiellen Inaktivierung von beispielsweise 115°C bei entsprechenden Haltezeiten. Der Temperaturverlauf in der Spirale entspricht in einer Variante dem zeitlich verzögerten Verlauf der Kammerinnentemperatur. In einer Variante wird die Sterilisation nach Einspeisung des Wasserdampfs aus dem Sofortdampferzeuger in die Kammer in der Kammer vervollständigt, d.h. der Wasserdampf in der Kammer einer Sterilisationsbedingung für einen ausreichenden Zeitraum unterworfen, so dass der SAL-Wert von 10⁻⁶ am Ende der Haltezeit erreicht wird. Die Spirale wird beispielsweise mittels einer Pumpe mit Speisewasser, z.B. aus einem Tank, gespeist. In einer Variante ist zwischen Spirale und Sofortdampferzeuger ein zwangsgesteuertes Ventil angeordnet. Nur in einem Verfahren mit Vorvakuum oder fraktionierten Vakuumverfahren ist aufgrund des Unterdrucks in der Kammer abhängig von der Bauart der Pumpe ein selbsttätiges Nachströmen von Wasser in den Sofortdampferzeuger möglich, welches sich mit einem zwangsgesteuerten Ventil vermeiden lässt. In einer alternativen Variante wird die Spirale in einem reinen Überdruckverfahren, auch Strömungsverfahren genannt, und ohne zwangsgesteuertes Ventil betrieben. In einem Beispiel ist zur Vermeidung einer Totstrecke und einer Vermischungsgefahr mit neuem Speisewasser im Bereich eines Speisewasserzugangs der Spirale in diesem Bereich ein Ventil, insbesondere ein Rückschlagventil angeordnet. Alternative Maßnahmen zur Vermeidung einer solchen Totstrecke und damit verbundenen Vermischungsgefahr sind der Fachperson bekannt und können ebenso Anwendung finden.

In einer weiteren Alternative wird das Wasser vor der Zufuhr zum Sofortdampferzeuger mittels eines Sterilfilters sterilisiert. Beispielsweise erfolgt die Sterilfiltration in einer Zuleitung von einem Tank für Speisewasser zum Sofortdampferzeuger. Alternativ oder zusätzlich erfolgt die Sterilfiltration im Bereich eines Zugangs zum Sofortdampferzeuger. In diesen beiden Varianten wird flüssiges Wasser mittels Sterilfiltration sterilisiert. Alternativ oder zusätzlich wird das im Sofortdampferzeuger erzeugte gasförmige Wasser mittels eines Sterilfilters vor der Bereitstellung in der Kammer sterilisiert. Beispielsweise ist im Bereich eines Ausgangs des Sofortdampferzeugers ein Sterilfilter angeordnet. Alternativ oder zusätzlich ist im Bereich der Zuleitung vom Sofortdampferzeuger zur Kammer ein Sterilfilter angeordnet. In diesen Varianten wird gasförmiges Wasser durch den bzw. die Sterilfilter geleitet, so dass dieser bzw. diese aufgrund der Dampfdurchströmung kontinuierlich sterilisiert werden. Auch im Bereich des Zugangs und des Ausgangs des Sofortdampferzeugers angeordnete Sterilfilter werden aufgrund der intensiven Wärmeeinwirkung durch den Sofortdampferzeuger kontinuierlich thermisch sterilisiert. In diesen Fällen ist ein Austausch der Sterilfilter erst am Ende der technischen Lebenszeit der Sterilfilter notwendig. Ein Austausch des Sterilfilters aufgrund einer Erschöpfung seiner hygienischen Kapazität ist lediglich bei einer Anordnung in einer Zuleitung von Speisewassertank zu Sofortdampferzeugung notwendig. Eine Anordnung von Sterilfiltern im Bereich eines Ausgangs des Sofortdampferzeugers oder in einer Zuleitung von Sofortdampferzeuger zur Kammer hat außerdem den weiteren Nutzen, dass Aerosoltropfen durch den Sterilfilter aufgehalten werden, die Qualität des von dem Sofortdampferzeuger generierten Wasserdampfs somit nicht nur in hygienischer Hinsicht, sondern auch in technischer Hinsicht erhöht wird.

Ein weiterer Gegenstand der vorliegenden Erfindung ist ein Autoklav zur Sterilisation von Sterilgut mit den Merkmalen des Anspruchs 11. Der Autoklav weist einen Sofortdampferzeuger zur Erzeugung von gasförmigem Wasser auf. Ferner weist der Autoklav eine Regelungseinheit zur Einstellung einer Sterilisationsbedingung in einer Kammer des Autoklaven für die Dauer einer Haltezeit auf. Die Kammer ist zur Aufnahme von Sterilgut geeignet und vorgesehen. Der Autoklav zeichnet sich dadurch aus, dass er ausgeführt ist, sämtliches während der Haltezeit in der Kammer befindliches Wasser zu sterilisieren, insbesondere so dass am Ende der Haltezeit in der Kammer ausschließlich steriles Wasser vorliegt. Mit dem Autoklav werden die Vorteile des erfindungsgemäßen Verfahrens zur Dampfsterilisation ebenfalls umgesetzt. Insbesondere wird vorliegend ein Autoklav mit einem Sofortdampferzeuger zur Verfügung gestellt, der hygienisch unbedenklich ist, weil das in der Kammer zur Sterilisation bereitgestellte Wasser spätestens am Ende der Haltezeit steril ist. Eine Kontamination der Kammer und von in der Kammer angeordnetem Sterilgut mit Mikroorganismen durch Sofortdampferzeugung wird somit vermieden.

Der Autoklav ist insbesondere ausgeführt, sämtliche im Hinblick auf das Verfahren zur Dampfsterilisation beschriebene Schritte auszuführen. Die den im Hinblick auf das Verfahren beschriebenen Merkmalen entsprechenden Vorrichtungsmerkmale stellen somit auch optionale Merkmale des Autoklaven dar und umgekehrt.

Gemäß einem Aspekt ist der Autoklav ausgeführt, sämtliches während der Haltezeit in der Kammer befindliches Wasser vor der Bereitstellung in der Kammer zu sterilisieren. Alternativ ist der Autoklav ausgeführt, sämtliches während der Haltezeit in der Kammer befindliches Wasser nach der Bereitstellung in der Kammer zu sterilisieren. In einer weiteren Alternative ist der Autoklav ausgeführt, sämtliches während der Haltezeit in der Kammer befindliches Wasser vor der Bereitstellung in der Kammer zu teilsterilisieren und nach der Bereitstellung in der Kammer vollständig zu sterilisieren. Der Autoklav weist beispielsweise eine Einheit zur Sterilisation oder Teilsterilisation des Wassers vor der Zuleitung in die Kammer auf.

In einer Variante ist der Sofortdampferzeuger außerhalb der Kammer angeordnet. Der Autoklav, insbesondere die Einheit zur Sterilisation oder Teilsterilisation des Wassers vor Zuleitung in die Kammer, weist in dieser Variante einen dem Sofortdampferzeuger vorgelagerten beheizbaren Vorlagebehälter zum Erhitzen von Wasser auf eine Temperatur oberhalb einer Sterilisationstemperatur auf. Die Temperatur oberhalb der Sterilisationstemperatur beträgt beispielsweise 150°C. Der Vorlagebehälter weist beispielsweise eine Heizung zum Erhitzen des Wassers in dem Vorlagebehälter auf. Der Vorlagebehälter ist somit geeignet, das in ihm angeordnete Wasser zu teilsterilisieren oder vollständig zu sterilisieren. Beispielsweise ist der Vorlagebehälter mit einem Tank für Speisewasser verbunden. In der Zuleitung ist in einer Variante eine Pumpe vorgesehen, mittels Speisewasser aus dem Tank in den Vorlagebehälter gepumpt werden kann. Ein Ausgang des Vorlagebehälters ist mit einem Zugang des Sofortdampferzeugers verbunden. In einer Variante ist in der Zuleitung von Vorlagebehälter zu Sofortdampferzeuger ein zwangsgesteuertes Ventil angeordnet. Im Vorlagebehälter entstehender Überdruck kann so genutzt werden, um das vorgeheizte Wasser mittels des Ventils in den Sofortdampferzeuger zu überführen. Der Vorlagebehälter ist insbesondere so ausgelegt, dass er das während der Haltezeit für ein Sterilisationsprogramm benötigte Wasser vorhalten, d.h. speichern, kann. Das in dem Sofortdampferzeuger generierte gasförmige Wasser wird über eine Zuleitung in der Kammer bereitgestellt.

In einer Variante ist der Sofortdampferzeuger außerhalb der Kammer angeordnet und der Autoklav, insbesondere die Einheit zur Sterilisation oder Teilsterilisation des Wassers vor Zuleitung in die Kammer, weist eine dem Sofortdampferzeuger vorgelagerte, in oder an der Kammer angeordnete Spirale zur Speicherung eines für einen Sterilisationsvorgang benötigten Wasservolumens auf. In der Spirale wird das durch sie geführte Wasser teilsterilisiert oder sterilisiert. Die Spirale ist so ausgelegt, dass sie das während einer Haltezeit für einen Sterilisationsvorgang benötigte Wasser speichern kann. Die Spirale wird in einer Variante mittels einer Pumpe mit Speisewasser aus einem Tank gespeist. Die Pumpe ist in einer Zuleitung zwischen einem Tank für Speisewasser und der Spirale angeordnet. Ein Ausgang der Spirale ist mit einem Zugang des Sofortdampferzeugers verbunden. In der Zuleitung zwischen Spirale und Sofortdampferzeuger ist beispielsweise ein zwangsgesteuertes Ventil angeordnet. Das in dem Sofortdampferzeuger generierte gasförmige Wasser wird über eine Zuleitung in die Kammer geleitet.

In einer weiteren Variante weist der Autoklav, insbesondere die Einheit zur Sterilisation oder Teilsterilisation des Wassers vor Zuleitung in die Kammer, ein mittels eines Ventils gegenüber der Kammer abgeriegeltes Dampfreservoir des Sofortdampferzeugers auf. Somit kann mit und in dem Sofortdampferzeuger gasförmiges Wasser auf Vorrat erzeugt werden. Über einen Ausgang des Sofortdampferzeugers, insbesondere dessen Dampfreservoir, kann das gasförmige Wasser über Stellvorgänge am Ventil in die Kammer geleitet werden. Während der Haltezeit wird somit kein neues Speisewasser benötigt. Der Autoklav arbeitet während der Haltezeit ausschließlich mit dem im Dampfreservoir gespeicherten gasförmigen Wasser.

In einer Variante weist der Autoklav, insbesondere die Einheit zur Sterilisation oder Teilsterilisation des Wassers vor Zuleitung in die Kammer, alternativ oder zusätzlich ein oder mehrere dem Sofortdampferzeuger vorgelagerte und/oder nachgelagerte Sterilfilter auf. Beispielsweise ist mindestens ein Sterilfilter in der Zuleitung von einem Tank für Speisewasser zum Sofortdampferzeuger angeordnet. Alternativ oder zusätzlich ist mindestens ein Sterilfilter im Bereich eines Zugangs, insbesondere im Zugang, zum Sofortdampferzeuger angeordnet. In beiden Fällen wird Speisewasser mittels Sterilfiltration sterilisiert. Alternativ oder zusätzlich ist mindestens ein Sterilfilter im Bereich eines Ausgangs, insbeondere im Ausgang, des Sofortdampferzeugers angeordnet. In einer weiteren Variante ist alternativ oder zusätzlich mindestens ein Sterilfilter in einer Zuleitung vom Sofortdampferzeuger zur Kammer angeordnet. In den beiden letzteren Fällen wird gasförmiges Wasser mittels Sterilfiltration sterilisiert.

Gemäß einem Aspekt ist der Autoklav ausgeführt, eine Einstellung der Sterilisationsbedingung in der Kammer für die Dauer der Haltezeit ausschließlich mittels Verdampfung des in der Kammer anfallenden Kondensats vorzunehmen oder der Autoklav ist ausgeführt, vor Beginn der Haltezeit Wasser in die Kammer zu speisen und eine Einstellung der Sterilisationsbedingung in der Kammer für die Dauer der Haltezeit ausschließlich mittels Verdampfung des vor Beginn der Haltezeit in die Kammer gespeisten Wassers und mittels Verdampfung des in der Kammer anfallenden Kondensats vorzunehmen. Hierzu weist der Autoklav eine Einheit zur Verdampfung des in der Kammer anfallenden Kondensats, oder des in der Kammer anfallenden Kondensats und des vor Beginn der Haltezeit in die Kammer gespeisten Wassers, auf. Diese erlaubt es dem Autoklaven, während der Haltezeit auf eine Zufuhr von neuem Wasser, insbesondere von frischem Speisewasser, zum Sofortdampferzeuger und somit zur Kammer zu verzichten. Das zur Einstellung der Sterilisationsbedingung in der Kammer verwendete Wasser unterliegt somit mindestens für die Dauer der Haltezeit der Sterilisationsbedingung und ist somit spätestens am Ende der Haltezeit steril.

In einer Variante weist der Autoklav, insbesondere die Einheit zur Verdampfung des in der Kammer anfallenden Kondensats, oder des in der Kammer anfallenden Kondensats und des vor Beginn der Haltezeit in die Kammer gespeisten Wassers, zur Verdampfung des in der Kammer anfallenden Kondensats, oder des in der Kammer anfallenden Kondensats und des vor Beginn der Haltezeit in die Kammer gespeisten Wassers, ein in der Kammer angeordnetes Heizelement auf. In einer alternativen Variante weist der Autoklav, insbesondere die Einheit zur Verdampfung des in der Kammer anfallenden Kondensats, oder des in der Kammer anfallenden Kondensats und des vor Beginn der Haltezeit in die Kammer gespeisten Wassers, zur Verdampfung des in der Kammer anfallenden Kondensats, oder des in der Kammer anfallenden Kondensats und des vor Beginn der Haltezeit in die Kammer gespeisten Wassers, den Sofortdampferzeuger und optional eine Pumpe zur Zufuhr von Kondensat bzw. von Kondensat und vor Beginn der Haltezeit in die Kammer gespeisten Wassers aus der Kammer in den Sofortdampferzeuger auf, wobei der Sofortdampferzeuger außerhalb der Kammer angeordnet ist. Die Einheit des zur Verdampfung des in der Kammer anfallenden Kondensats, oder des in der Kammer anfallenden Kondensats und des vor Beginn der Haltezeit in die Kammer gespeisten Wassers, umfasst in dieser Variante somit den außerhalb der Kammer angeordneten Sofortdampferzeuger sowie optional eine Pumpe zur Zufuhr von Kondensat, bzw. von in der Kammer anfallenden Kondensats und von vor Beginn der Haltezeit in die Kammer gespeisten Wassers, aus der Kammer in den Sofortdampferzeuger. In einer weiteren Variante weist der Autoklav, insbesondere die Einheit zur Verdampfung des in der Kammer anfallenden Kondensats, oder des in der Kammer anfallenden Kondensats und des vor Beginn der Haltezeit in die Kammer gespeisten Wassers, zur Verdampfung des in der Kammer anfallenden Kondensats, oder des in der Kammer anfallenden Kondensats und des vor Beginn der Haltezeit in die Kammer gespeisten Wassers, den Sofortdampferzeuger auf, wobei der Sofortdampferzeuger in der Kammer angeordnet ist. Die Einheit des zur Verdampfung des in der Kammer anfallenden Kondensats, oder des in der Kammer anfallenden Kondensats und des vor Beginn der Haltezeit in die Kammer gespeisten Wassers, umfasst in dieser Variante somit den in der Kammer angeordneten Sofortdampferzeuger.

Einzelheiten von Aspekten der vorliegend beanspruchten Erfindung werden nachfolgend anhand von Ausführungsbeispielen und Figuren näher erläutert. Es zeigen:

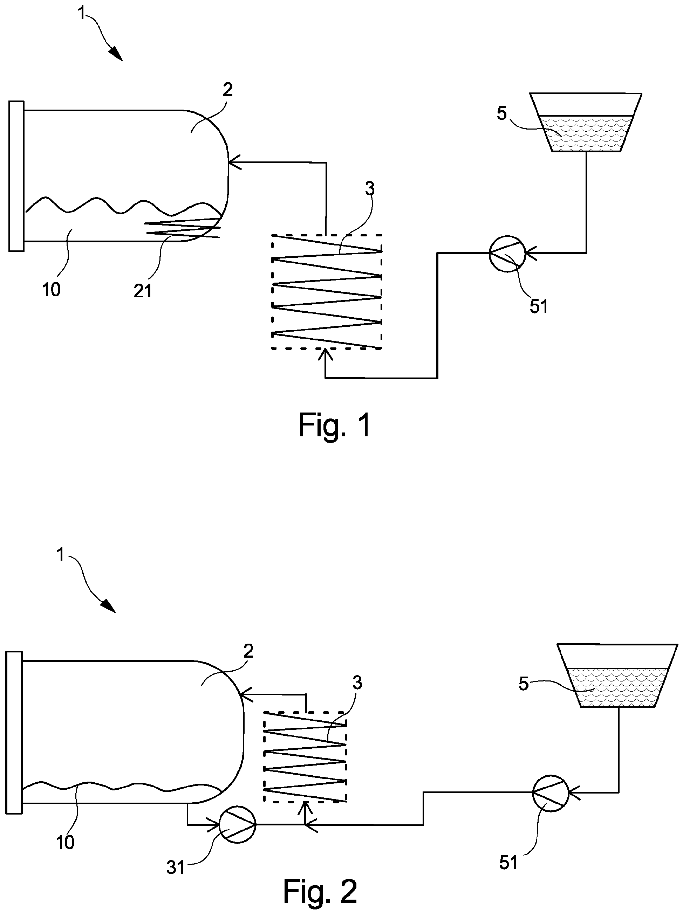

- Figur 1: eine Darstellung eines Verfahrens zur Dampfsterilisation mit einem Autoklaven sowie ein entsprechender Autoklav gemäß einer Variante der vorliegenden Erfindung, wobei sämtliches in der Kammer während der Haltezeit bereitgestelltes Wasser nach der Bereitstellung in der Kammer sterilisiert wird;
- Figur 2: eine Darstellung eines Verfahrens zur Dampfsterilisation mit einem Autoklaven sowie ein entsprechender Autoklav gemäß einer alternativen Variante der vorliegenden Erfindung, wobei sämtliches in der Kammer während der Haltezeit bereitgestelltes Wasser nach der Bereitstellung in der Kammer sterilisiert wird;

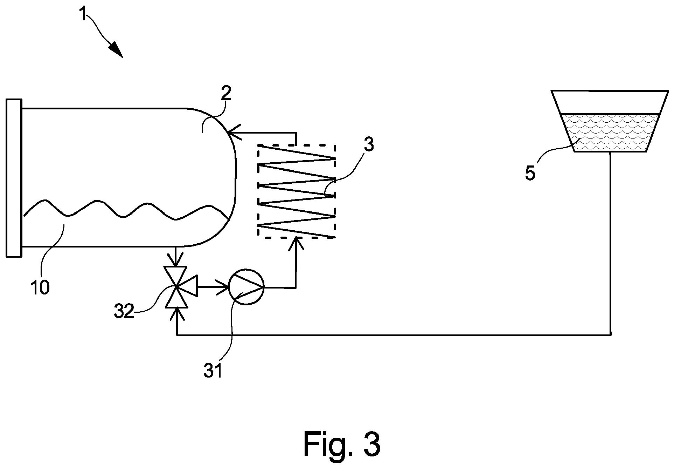

- Figur 3: eine Darstellung eines Verfahrens zur Dampfsterilisation mit einem Autoklaven sowie ein entsprechender Autoklav gemäß einer alternativen Variante der vorliegenden Erfindung, wobei sämtliches in der Kammer während der Haltezeit bereitgestelltes Wasser nach der Bereitstellung in der Kammer sterilisiert wird;

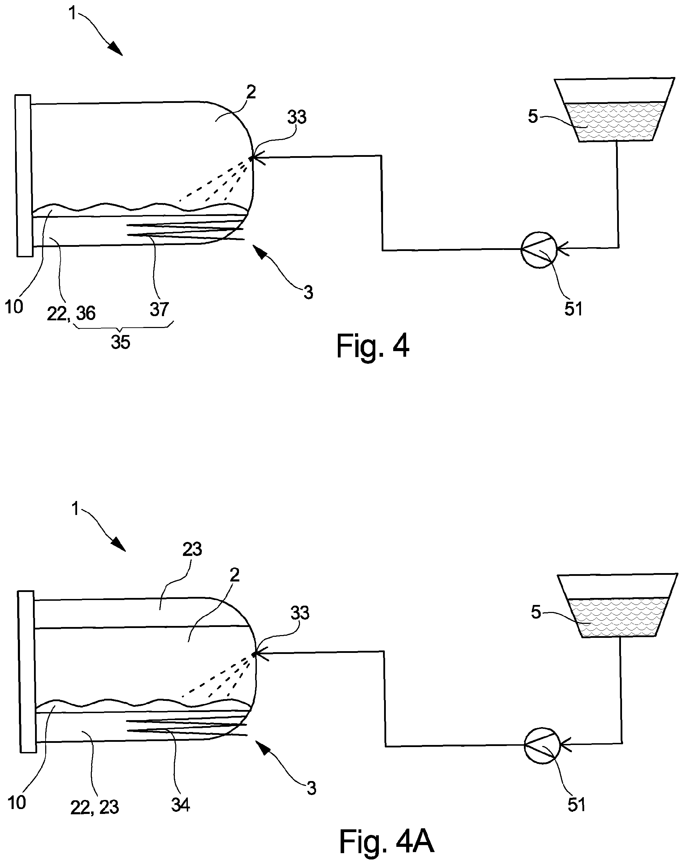

- Figur 4: eine Darstellung eines Verfahrens zur Dampfsterilisation mit einem Autoklaven sowie ein entsprechender Autoklav gemäß einer alternativen Variante der vorliegenden Erfindung, wobei sämtliches in der Kammer während der Haltezeit bereitgestelltes Wasser nach der Bereitstellung in der Kammer sterilisiert wird;
- Figur 4a: eine Darstellung eines Verfahrens zur Dampfsterilisation mit einem Autoklaven sowie ein entsprechender Autoklav in einer weiteren Variante der vorliegenden Erfindung;

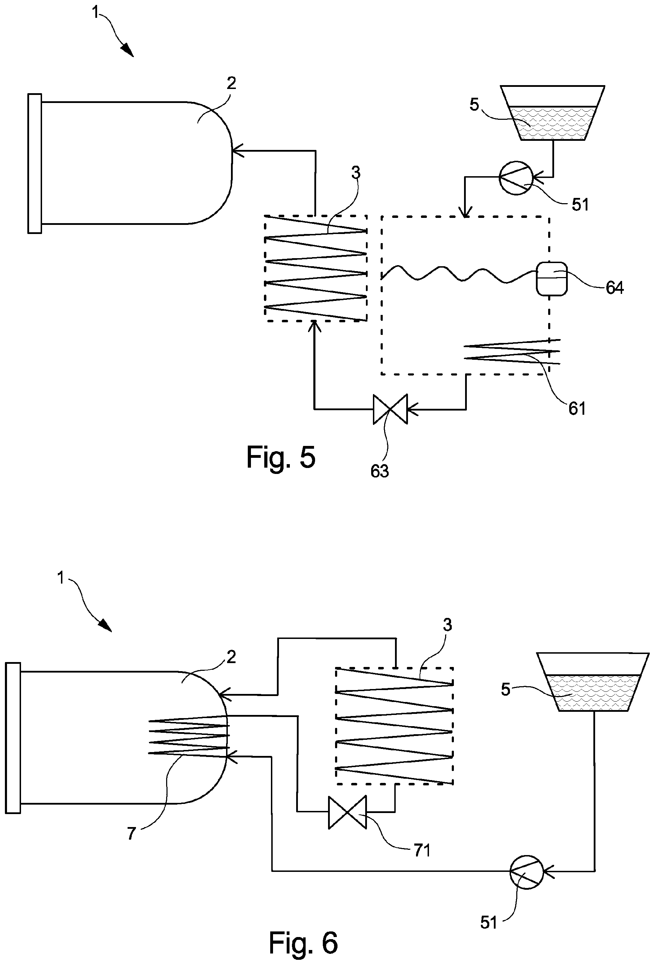

- Figur 5: eine Darstellung eines Verfahrens zur Dampfsterilisation mit einem Autoklaven sowie ein entsprechender Autoklav gemäß einer Variante der vorliegenden Erfindung, wobei sämtliches in der Kammer während der Haltezeit bereitgestelltes Wasser vor der Bereitstellung in der Kammer sterilisiert wird oder vor der Bereitstellung in der Kammer teilsterilisiert und nach der Bereitstellung in der Kammer vollständig sterilisiert wird;
- Figur 6: eine Darstellung eines Verfahrens zur Dampfsterilisation mit einem Autoklaven sowie ein entsprechender Autoklav gemäß einer alternativen Variante der vorliegenden Erfindung, wobei sämtliches in der Kammer während der Haltezeit bereitgestelltes Wasser vor der Bereitstellung in der Kammer sterilisiert wird oder vor der Bereitstellung in der Kammer teilsterilisiert und nach der Bereitstellung in der Kammer vollständig sterilisiert wird;

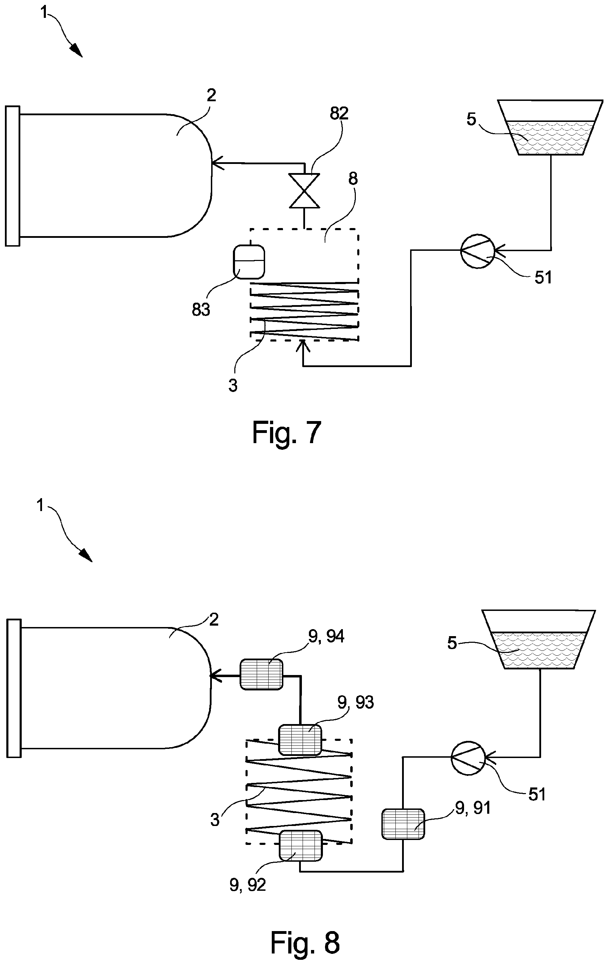

- Figur 7: eine Darstellung eines Verfahrens zur Dampfsterilisation mit einem Autoklaven sowie ein entsprechender Autoklav gemäß einer alternativen Variante der vorliegenden Erfindung, wobei sämtliches in der Kammer während der Haltezeit bereitgestelltes Wasser vor der Bereitstellung in der Kammer sterilisiert wird; und
- Figur 8: eine Darstellung eines Verfahrens zur Dampfsterilisation mit einem Autoklaven sowie ein entsprechender Autoklav gemäß einer alternativen Variante der vorliegenden Erfindung, wobei sämtliches in der Kammer während der Haltezeit bereitgestelltes Wasser vor der Bereitstellung in der Kammer sterilisiert wird.

Figur 1 illustriert ein Verfahren zur Dampfsterilisation in einem Autoklaven 1 sowie einen entsprechenden Autoklaven 1. Bei der Dampfsterilisation wird die Anzahl der aktiven, d.h. lebensfähigen, Mikroorganismen unter einen normativ definierten Schwellenwert gebracht. Gemäß DIN EN 556 gilt ein Medizinprodukt beispielsweise als steril, wenn die theoretische Wahrscheinlichkeit, einen lebensfähigen Mikroorganismus zu finden, kleiner als 1:10⁶ ist. Dies entspricht einem SAL-Wert von 10⁻⁶. Mikroorganismen werden bei der Dampfsterilisation unschädlich gemacht, indem sie über eine gewissen Zeitdauer, die sogenannte Haltezeit, gasförmigem Wasser, d.h. Wasserdampf, bei einer bestimmten Sterilisationsbedingung, beispielsweise einer Sterilisationstemperatur bei Sattdampfbedingungen, ausgesetzt werden. Dabei hängt die Haltezeit von der gewählten Sterilisationsbedingung, insbesondere der Sterilisationstemperatur bei Sattdampfbedingungen, ab. Normen EN 13060 und EN 285 definieren, wie bereits erwähnt, solche Haltezeit-Sterilisationsbedingungs-Beziehungen. In einem Autoklaven 1 wird zur Erzeugung von gasförmigem Wasser, d.h. Wasserdampf, flüssiges Wasser, sogenanntes Speisewasser, in einem Tank 5 zur Verfügung gestellt. Das Speisewasser wird, wie hier dargestellt, über eine Pumpe 51 einem Dampferzeuger zugeführt. Vorliegend beschriebener Autoklav 1 weist zur Erzeugung von gasförmigem Wasser einen Sofortdampferzeuger 3 auf. Ein Sofortdampferzeuger 3 ermöglicht eine quasi instantane Verdampfung des zugeführten Speisewassers. Dies macht den Sofortdampferzeuger 3 aus Effizienz- und Kostengründen attraktiv. Während der Haltezeit wird das so erzeugte gasförmige Wasser, d.h. der im Sofortdampferzeuger 3 generierte Wasserdampf, in einer Kammer 2 des Autoklaven 1 bereit gestellt, um eine Sterilisation von in der Kammer 2 angeordnetem Sterilgut zu bewirken. Hierzu wird durch eine entsprechende Regelungseinheit der Kammer 2 für die Dauer einer Haltezeit eine Sterilisationsbedingung eingestellt. Beispielsweise wird bei Sattdampfbedingungen für 15 Minuten eine Temperatur von 121°C eingestellt. Die Einstellung der Sterilisationsbedingung für die Dauer der Haltezeit erfordert beim Einsatz eines Sofortdampferzeugers 3 ein mehrmaliges Nachspeisen von gasförmigem Wasser in die Kammer 2. Um eine Kontamination der Kammer 2 und von darin angeordnetem Sterilgut durch das Nachspeisen zu verhindern, ist der Autoklav 1 ausgeführt, sämtliches in der Kammer 2 während der Haltezeit bereitgestelltes Wasser, insbesondere nach der Bereitstellung in der Kammer 2, zu sterilisieren, so dass am Ende der Haltezeit in der Kammer 2 ausschließlich steriles Wasser vorliegt. Insbesondere ist der Autoklav 1 ausgeführt, die Einstellung der Sterilisationsbedingung mittels der Regelungseinheit ausschließlich durch eine Verdampfung von in der Kammer 2 anfallendem Kondensat 10 vorzunehmen. In einer alternativen Variante ist der Autoklav 1 ausgeführt, die Einstellung der Sterilisationsbedingung mittels der Regelungseinheit ausschließlich durch eine Verdampfung von vor Beginn der Haltezeit in die Kammer 2 gespeistem flüssigem Wasser und durch eine Verdampfung von in der Kammer 2 anfallendem Kondensat 10 vorzunehmen. Insbesondere wird durch das Einspeisen von Wasser, insbesondere flüssigem Wasser, vor Beginn der Haltezeit sichergestellt, dass während der Haltezeit in der Kammer 2 genügend Wasser zur Verfügung steht, um die Sterilisationsbedingung einzustellen und aufrechtzuerhalten. Ein Verdampfen und Nachspeisen mit frischem Speisewasser aus dem Tank 5 kann so in beiden Varianten unterbleiben. Nach Beginn der Haltezeit wird der Kammer 2 kein neues Wasser hinzugefügt. Es wird lediglich das anfallende Kondensat 10 neu verdampft. Hierzu ist eine Einheit zur Verdampfung des in der Kammer 2 anfallenden Kondensats vorgesehen, die vorliegend ein in der Kammer 2 angeordnetes Heizelement 21 umfasst. Das Heizelement 21 wird mittels der Regelungseinheit derart gesteuert, dass eine Sterilisationsbedingung in der Kammer 2 für die Dauer der Haltezeit herrscht. Sämtliches während der Haltezeit in der Kammer 2 anfallendes Wasser ist somit für die Dauer der Haltezeit der Sterilisationsbedingung unterworfen. Somit ist es spätestens am Ende der Haltezeit steril. Es wird somit ein Verfahren zur Dampfsterilisation in einem Autoklaven 1 sowie eine entsprechender Autoklav zur Verfügung gestellt, der die Vorteile eines Sofortdampferzeugers 3 mit einer hygienisch einwandfreien Prozessführung vereint.

Figur 2 illustriert ein Verfahren zur Dampfsterilisation in einem Autoklaven 1 sowie einen entsprechenden Autoklaven 1 gemäß einer weiteren Variante. Auch in dieser Variante wird eine hygienische Prozessführung erreicht, indem sämtliches in der Kammer 2 während der Haltezeit bereitgestelltes Wasser, insbesondere nach der Bereitstellung in der Kammer 2, sterilisiert wird. Dies erfolgt, wie bei dem Verfahren und dem Autoklaven 1 gemäß der in Figur 1 gezeigten Variante, ebenfalls dadurch, dass die Sterilisationsbedingung während der Haltezeit ausschließlich durch Verdampfung von in der Kammer 2 anfallendem Kondensat 10 oder ausschließlich durch Verdampfung von vor Beginn der Haltezeit in die Kammer 2 gespeistem Wasser sowie durch Verdampfung von in der Kammer 2 anfallendem Kondensat 10 eingestellt wird. Hierzu ist eine Einheit zur Verdampfung des in der Kammer 2 anfallenden Kondensats, insbesondere des in der Kammer anfallenden Kondensats und des vor Beginn der Haltezeit in die Kammer gespeisten Wassers, vorgesehen, die vorliegend eine Pumpe 31 und den außerhalb der Kammer 2 angeordneten Sofortdampferzeuger 3 umfasst. Im Gegensatz zur Variante der Figur 1 erfolgt in der hier dargestellten Variante die Verdampfung des Kondensats 10, insbesondere des in der Kammer anfallenden Kondensats und des vor Beginn der Haltezeit in die Kammer gespeisten Wassers, nicht in der Kammer 2 selbst. Vielmehr wird das Kondensat 10 bzw. das Kondensat 10 sowie das in die Kammer 2 vor Beginn der Haltezeit gespeiste Wasser, beispielsweise durch einen zum Abführen von Kondensat 10 aus der Kammer 2 vorgesehenen Anschluss, aus der Kammer 2 in den Sofortdampferzeuger 3 gepumpt. Hierzu ist eine Pumpe 31 vorgesehen. In dem Sofortdampferzeuger 3 wird das Kondensat 10 bzw. das Kondensat 10 sowie das in die Kammer 2 vor Beginn der Haltezeit gespeiste Wasser verdampft und der Kammer 2 erneut zugeführt. Ein Nachspeisen des Sofortdampferzeugers 3 mit frischem Speisewasser ist nicht notwendig, so dass am Ende der Haltezeit sämtliches während der Haltezeit in der Kammer 2 anfallendes Wasser für die Dauer der Haltezeit der Sterilisationsbedingung unterworfen war, somit am Ende der Haltezeit steril ist. Zur Vermeidung von Totstrecken und einer Vermischung des Kondensats 10 bzw. das Kondensat 10 sowie das in die Kammer 2 vor Beginn der Haltezeit gespeiste Wasser mit Speisewasser, ist optional ein Rückschlagventil nah am aus Tank 5 und Pumpe 51 gebildeten Wasserkreislauf vorgesehen. Alternativ ist die Pumpe 31 mit einem integrierten Rückschlagventil nah am Wasserkreislauf angeordnet.

Figur 3 zeigt ein Verfahren zur Dampfsterilisation in einem Autoklaven 1 sowie einen entsprechenden Autoklaven 1 in einer gegenüber der Variante der Figur 2 leicht abgewandelten Variante. Im Folgenden werden somit nur die Abwandlungen gegenüber der im Hinblick auf Figur 2 dargestellten Variante beschrieben. Die hier dargestellte Variante verzichtet gegenüber der Variante der Figur 2 auf die Pumpe 51 für Speisewasser. Stattdessen wird sowohl das in der Kammer 2 anfallende Kondensat 10 bzw. das Kondensat 10 sowie das in die Kammer 2 vor Beginn der Haltezeit gespeiste Wasser als auch vor Beginn der Haltezeit zugeführtes Speisewasser über ein Mehrwege-Ventil 32, dargestellt beispielsweise ein 3/2-Wege-Ventil, einer Pumpe 31 und durch diese dem Sofortdampferzeuger 3 zugeführt. Eine Zufuhr von Speisewasser aus dem Tank 5 zum Sofortdampferzeuger 3 erfolgt lediglich zu Beginn der Haltezeit. Während der Haltezeit erfolgt die Einstellung einer Sterilisationsbedingung lediglich durch die Verdampfung von in der Kammer 2 anfallendem Kondensat 10, insbesondere durch Verdampfung des in der Kammer anfallenden Kondensats und des vor Beginn der Haltezeit in die Kammer gespeisten Wassers, das mittels der Pumpe 31 dem Sofortdampferzeuger 3 zugeführt und hier verdampft wird. Um die Sterilisationsbedingung für die Dauer der Haltezeit zu gewährleisten, ist beispielsweise der aus dem Mehrwege-Ventil 32, der Pumpe 31 und dem Sofortdampferzeuger 3 bestehende Kreislauf, mit kurzen Rohrlängen und thermisch isoliert ausgeführt. In einer Variante wird die Haltezeit geringfügig verlängert.

In Figur 4 ist ein Verfahren zur Dampfsterilisation in einem Autoklaven 1 sowie ein entsprechender Autoklav 1 in einer weiteren Variante dargestellt. Auch hier wird sämtliches während der Haltezeit in der Kammer 2 befindliches Wasser sterilisiert. Insbesondere wird das Wasser nach der Bereitstellung in der Kammer 2 sterilisiert. Hierzu ist der Sofortdampferzeuger 3 in der Kammer 2 angeordnet, so dass in der Kammer 2 anfallendes Kondensat 10 oder das in der Kammer 2 anfallende Kondensat 10 und das in die Kammer 2 vor Beginn der Haltezeit gespeiste Wasser im Sofortdampferzeuger 3 in der Kammer 2 verdampft wird und so eine Sterilisationsbedingung während der Haltezeit in der Kammer 2 eingestellt wird. Der Sofortdampferzeuger 3 umfasst einen in der Kammer 2 angeordneten Heizkörper 35, insbesondere eine in einem Heizblock 36 angeordnete Heizspirale 37 oder eine Heizpatrone (nicht dargestellt). Die Heizspirale 37 erhitzt den Heizblock 36. Auf den Heizkörper 35 auftreffendes, insbesondere flüssiges, Wasser wird somit instantan verdampft. Flüssiges Wasser wird über eine Düse 33 in die Kammer 2 gespritzt. Mittels der Düse 33 wird das flüssige Wasser auf den Heizkörper 35 gespritzt und dort verdampft. Die Einheit zur Verdampfung des in der Kammer 2 anfallenden Kondensats, oder des in der Kammer anfallenden Kondensats und des vor Beginn der Haltezeit in die Kammer gespeisten Wassers, umfasst vorliegend somit die Düse 33 und den in der Kammer 2 angeordneten Sofortdampferzeuger 3. Der Heizkörper 35 ist Bereich des Bodens 22 der Kammer 2 angeordnet, so dass in der Kammer 2 anfallendes Kondensat 10 bzw. das Kondensat 10 sowie das in die Kammer 2 vor Beginn der Haltezeit gespeiste Wasser auf den Sofortdampferzeuger 3 gelangt und von diesem verdampft wird. Beispielsweise bildet der Heizblock 36 den Boden 22 der Kammer 2. Ein Nachspeisen von Speisewasser aus dem Tank 5 während der Haltezeit in die Kammer 2 bzw. auf den Sofortdampferzeuger 3 erfolgt nicht. Sämtliches während der Haltezeit in der Kammer 2 anfallendes Wasser ist somit für die Dauer der Haltezeit der Sterilisationsbedingung ausgesetzt und somit spätestens am Ende der Haltezeit steril. Hiermit wird ein besonders einfacher Aufbau eines Autoklaven 1 mit Sofortdampferzeuger 3 und hygienischer Prozessführung zur Verfügung gestellt.

In Figur 4a ist ein Verfahren zur Dampfsterilisation mit einem Autoklaven 1 und ein entsprechender Autoklav 1 gemäß der in einer Abwandlung der in Figur 4 dargestellten Variante näher erläutert. Der Autoklav 1 weist eine Kammer 2 auf sowie einen in der Kammer 2 angeordneten Sofortdampferzeuger 3 auf. Der Sofortdampferzeuger 3 umfasst Teile der Kammerwandung 23 oder die gesamte Kammerwandung 23. Insbesondere umfasst der Sofortdampferzeuger 3 den Boden 22 der Kammer 2. Im Boden 22 ist in der Kammerwandung 23 ein Heizelement 34, insbesondere eine Heizpatrone, angeordnet. Das Heizelement 34, insbesondere die Heizpatrone, erhitzt die Kammerwandung 23, insbesondere den Kammerboden 22. Auf die Kammerwandung 23, insbesondere den Kammerboden 22, auftreffendes Wasser, kann so instantan verdampft werden. Insbesondere wird flüssiges Wasser über eine Düse 33 in die Kammer 2 gespritzt. Mittels der Düse 33 wird das flüssige Wasser auf eine Kammerwandung 23, insbesondere einen Boden 22 der Kammerwandung 23, gespritzt und dort verdampft. Die Einheit zur Verdampfung des in der Kammer 2 anfallenden Kondensats, oder des in der Kammer anfallenden Kondensats und des vor Beginn der Haltezeit in die Kammer gespeisten Wassers, umfasst vorliegend somit die Düse 33 und den in der Kammer 2 angeordneten Sofortdampferzeuger 3. Die Kammer 2 selbst ist ein stranggepresster Kessel aus einem wärmeleitfähigen Metall, beispielsweise ein stranggepresster Aluminiumkessel Figur 5 illustriert ein Verfahren zur Dampfsterilisation in einem Autoklaven 1 und einen entsprechenden Autoklaven 1 in einer weiteren Variante. Auch in dieser Variante wird sämtliches während der Haltezeit in der Kammer 2 befindliches Wasser sterilisiert. Im Gegensatz zu den in den Figuren 1 bis 4 dargestellten Varianten, wird gemäß der in Figur 5 dargestellten Variante sämtliches während der Haltezeit in der Kammer 2 befindliches Wasser vor der Bereitstellung in der Kammer 2 sterilisiert oder vor der Bereitstellung in der Kammer 2 zumindest teilsterilisiert und nach der Bereitstellung in der Kammer 2 vollständig sterilisiert. In der vorliegenden Variante wird eine Sterilisationsbedingung in der Kammer 2 durch ein Nachspeisen von gasförmigem Wasser in die Kammer 2 während der Haltezeit, beispielsweise mittels der Regelungseinheit, eingestellt. Das Wasser wird dem Sofortdampferzeuger 3 beispielsweise aus einem Tank 5 für Speisewasser, insbesondere mittels einer Pumpe 51, zugeführt. Vor der Zufuhr zum Sofortdampferzeuger 3 wird das Wasser sterilisiert oder teilsterilisiert. Hierzu ist eine Einheit zur Sterilisation oder Teilsterilisation des Wassers vor der Zuleitung in die Kammer 2 vorgesehen. Diese weist vorliegend einen dem Sofortdampferzeuger 3 vorgelagerten Vorlagebehälter 6 auf. Dieser verfügt über ein Heizelement 61. Mittels des Heizelements 61 wird Wasser in dem Vorlagebehälter 6 auf eine Temperatur oberhalb einer Sterilisationstemperatur erhitzt. Die Sterilisationstemperatur ist beispielsweise durch die in der Kammer 2 eingestellte Sterilisationsbedingung vorgegeben. Beispielsweise wird das Wasser in dem Vorlagebehälter 6 auf eine Temperatur von 150°C erhitzt. Der hierdurch entstehende Überdruck wird in einer Variante genutzt, um das vorgeheizte Wasser mit Hilfe eines zwangsgesteuerten Ventils 63 in den Sofortdampferzeuger 3 zu überführen. Dabei ist der Vorlagebehälter 6 so ausgeführt, dass er das Wasservolumen, das während einer Haltezeit für ein Sterilisationsprogramm benötigt wird, speichern kann. Das in den Sofortdampferzeuger 3 gelangende Wasser wurde somit bereits in dem Vorlagebehälter 6 sterilisiert, oder zumindest teilsterilisiert. Erfolgt in Vorlagebehälter 6 lediglich eine Teilsterilisation des Wassers, wird das Wasser in der Kammer 2, beispielsweise indem es für die verbleibende Dauer der Haltezeit der Sterilisationsbedingung in der Kammer 2 ausgesetzt wird, vollständig sterilisiert. In den der Haltezeit vorangehenden Entlüftungs- und Dampfeinlassphasen kann im Vorlagebehälter 6 eine Temperatur unterhalb der Sterilisationstemperatur vorliegen. In diesen Phasen wird das Speisewasser aus dem Tank 5 mittels der Pumpe 51 durch den Vorlagebehälter 6 hindurch in den Sofortdampferzeuger 3 geleitet oder mittels eines Überdrucks im Vorlagebehälter 6 durch diesen in den Sofortdampferzeuger 3 geführt werden. Zur Vermeidung einer Totstrecke und einer Vermischungsgefahr von im Vorlagebehälter vorliegendem Wasser mit aus dem Tank 5 stammenden Speisewasser, ist optional im Bereich des Zugangs des Vorlagebehälters 6 ein Rückschlagventil angeordnet.

Figur 6 zeigt ein Verfahren zur Dampfsterilisation in einem Autoklaven 1 und einen entsprechenden Autoklaven 1 in einer weiteren Variante. Dabei wird auch in dieser Variante, wie in der in Bezug auf Figur 5 beschriebenen Variante, das Wasser vor der Zufuhr zum Sofortdampferzeuger 3 sterilisiert oder teilsterilisiert. Hierzu ist eine Einheit zur Sterilisation oder Teilsterilisation des Wassers vor der Zuleitung in die Kammer 2 vorgesehen. Diese weist vorliegend eine dem Sofortdampferzeuger 3 vorgelagerte, in der Kammer 2 angeordnete Spirale 7 auf. Die Spirale 7 ist in einer alternativen Variante an der Kammer 2 angeordnet. Wichtig ist, dass das die Spirale durchströmende Wasser für die Dauer der Haltezeit der Sterilisationsbedingung in der Kammer 2 unterworfen ist. Die Spirale ist so ausgeführt, dass sie ein für einen Sterilisationsvorgang während der Haltezeit benötigtes Wasservolumen speichern kann. So wird in dieser Variante dem Sofortdampferzeuger 3 zwar ebenfalls frisches Speisewasser zur Einstellung einer Sterilisationsbedingung in der Kammer 2 zugeführt, aber auch in dieser Variante ist das Wasser bereits sterilisiert oder zumindest teilsterilisiert, wenn es den Sofortdampferzeuger 3 erreicht. Wird das Wasser in der Spirale 7 lediglich teilsterilisiert, beispielsweise weil eine Sterilisationsbedingung in der Spirale 7 nicht für die Dauer der Haltezeit erreicht wird, erfolgt eine vollständige Sterilisation in der Kammer 2. Der Speisevorgang aus dem Tank 5 erfolgt mittels einer Pumpe 51 und eines zwischen Spirale 7 und Sofortdampferzeuger 3 angeordneten zwangsgesteuerten Ventils 71. Wird der Autoklav 1 in einem reinen Überdruckverfahren betrieben, kann auf das Ventil 71 verzichtet werden. Zur Vermeidung einer Totstrecke und einer Vermischungsgefahr von in der Spirale befindlichem Wasser mit Speisewasser aus dem Tank 5 ist in einer Variante ein Rückschlagventil im Bereich des Zugangs der Spirale 7 angeordnet. Auch in dieser Variante wird eine Autoklav 1 mit einem Sofortdampferzeuger 3 hygienisch betrieben.

In Figur 7 ist ein Verfahren zur Dampfsterilisation in einem Autoklaven 1 und ein entsprechender Autoklav 1 in einer weiteren Variante illustriert. Dabei wird in dieser Variante sämtliches während der Haltezeit in der Kammer 2 bereitgestelltes Wasser vor der Bereitstellung in der Kammer 2 sterilisiert. Gleichzeitig erfolgt eine Einstellung der Sterilisationsbedingung in der Kammer 2 während der Haltezeit auch durch ein Nachspeisen von gasförmigem Wasser in die Kammer 2. Hierzu ist eine Einheit zur Sterilisation oder Teilsterilisation des Wassers vor der Zuleitung in die Kammer 2 vorgesehen. Diese umfasst den Sofortdampferzeuger 3 mit einem gegenüber der Kammer 2 abgeriegelten Dampfreservoir 8. Im Sofortdampferzeuger 3 erzeugtes gasförmiges Wasser kann so im Dampfreservoir 8 gespeichert werden und während der Haltezeit zur Einstellung der Sterilisationsbedingung in die Kammer 2 geleitet werden. Die dargestellte Variante stellt somit eine Mischform aus einem klassischen Sofortdampferzeuger 3 und einem abgeriegelten Dampferzeuger dar. Das Dampfreservoir 8 wird beispielsweise mittels eines Ventils 82 von der Kammer 2 abgeriegelt. Im Sofortdampferzeuger 3 wird beispielsweise während einer Entlüftungs- und Aufheizphase gasförmiges Wasser erzeugt und in dem Dampfreservoir 8 gespeichert. Während der Haltezeit wird zur Einstellung der Sterilisationsbedingung in der Kammer 2 durch Stellvorgänge am Ventil 82 gasförmiges Wasser aus dem Dampfreservoir 8 in die Kammer 2 geleitet. Während der Haltezeit wird somit kein frisches Speisewasser aus dem Tank 5 in den Sofortdampferzeuger 3 und somit in die Kammer 2 geleitet. Sämtliches während der Haltezeit in der Kammer 2 bereitgestelltes Wasser wurde in dem Dampfreservoir 8, d.h. vor Bereitstellung in der Kammer 2 sterilisiert. Somit wird eine hygienische Dampfsterilisation in einem Autoklaven 1 mit Sofortdampferzeuger 3 ermöglicht. Zur Vermeidung einer Totstrecke und einer Vermischungsgefahr von in dem Sofortdampferzeuger 3 befindlichem Wasser mit Speisewasser aus dem Tank 5 ist in einer Variante am Zugang zum Sofortdampferzeuger 3 ein Rückschlagventil angeordnet.

Figur 8 zeigt ein Verfahren zur Dampfsterilisation in einem Autoklaven 1 und einen entsprechenden Autoklaven 1 in einer weiteren Variante. In dieser Variante wird sämtliches in der Kammer 2 während der Haltezeit bereitgestelltes Wasser vor Bereitstellung in der Kammer 2 sterilisiert. Dabei erfolgt eine Einstellung einer Sterilisationsbedingung in der Kammer 2 auch durch Nachspeisen von Speisewasser aus dem Tank 5 in den Sofortdampferzeuger 3. Die Einheit zur Sterilisation oder Teilsterilisation des Wassers vor der Zuleitung in die Kammer 2 umfasst vorliegend mindestens einen Sterilfilter 9. Die Sterilisation vor Bereitstellung in der Kammer 2 erfolgt durch mindestens einen Sterilfilter 9. Je nach Variante sind ein oder mehrere Sterilfilter 9 vorgesehen. Ein oder mehrere der in dieser Figur 8 dargestellten Sterilfilter 91 bis 94 können in einer Variante des Autoklaven 1 angeordnet sein. Beispielsweise wird das, insbesondere flüssige, Wasser vor der Zufuhr zum Sofortdampferzeuger 3 mittels mindestens eines Sterilfilters 91, 92 sterilisiert. Mindestens ein Sterilfilter 91 ist zum Beispiel in einer Zuleitung für Speisewasser vom Tank 5 zum Sofortdampferzeuger 3 angeordnet. Da ein solcher Sterilfilter 91 nicht von der Wärmeleistung des Sofortdampferzeugers 3 profitiert, muss er bei Erreichen seiner hygienischen Kapazität ausgetauscht werden. Alternativ oder zusätzlich ist mindestens ein Sterilfilter 92 im Bereich eines Zugangs, insbesondere im Zugang, des Sofortdampferzeugers 3 angeordnet. Ein solcher Sterilfilter 92 profitiert von der von dem Sofortdampferzeuger 3 abgegebenen Wärme und wird durch diese kontinuierlich thermisch sterilisiert, so dass ein Austausch des Sterilfilters 92 erst am Ende der technischen Lebenszeit des Sterilfilters 92 notwendig wird. Alternativ oder zusätzlich ist mindestens ein Sterilfilter 93, 94 vorgesehen, mittels dessen das im Sofortdampferzeuger 3 generierte gasförmige Wasser vor seiner Bereitstellung in der Kammer 2 sterilisiert wird. Beispielsweise ist ein Sterilfilter 93 im Bereich des Ausgang des Sofortdampferzeugers 3, insbesondere im Ausgang des Sofortdampferzeugers 3, angeordnet. Ein solcher Sterilfilter 93 wird durch die Wärmeeinwirkung des Sofortdampferzeugers 3 kontinuierlich thermisch sterilisiert. Darüber hinaus wird das durchströmende gasförmige Wasser sterilisiert. Da der Sterilfilter 93 auch etwaige im gasförmigen Wasser verbliebene Aerosoltropfen aufhält, wird durch den Sterilfilter 93 die Qualität des gasförmigen Wassers nicht nur hygienisch sondern auch technisch verbessert. Dasselbe gilt für einen alternativ oder zusätzlich in der Zuleitung zwischen Sofortdampferzeuger 3 und Kammer 2 angeordneten mindestens einen Sterilfilter 94. Ein solcher Sterilfilter 94 wird aufgrund der Durchströmung mit gasförmigem Wasser kontinuierlich sterilisiert und verbessert ebenfalls die technische Qualität des gasförmigen Wassers vor dessen Zufuhr in die Kammer 2. So wird auch mit dieser Variante eine hygienische Dampfsterilisation mit Sofortdampferzeugung ermöglicht.

### Bezugszeichenliste

- 1: Autoklav
- 2: Kammer
- 21: Heizelement
- 22: Kammerboden
- 23: Kammerwandung
- 3: Sofortdampferzeuger
- 31: Pumpe
- 32: Mehrwege-Ventil
- 33: Düse
- 34: Heizelement
- 35: Heizkörper
- 36: Heizblock
- 37: Heizspirale
- 5: Speisewassertank
- 51: Pumpe
- 6: Vorlagebehälter
- 61: Heizelement
- 63: Ventil
- 64: Niveauregelung
- 7: Spirale
- 71: Ventil
- 8: Dampfreservoir
- 82: Ventil
- 83: Niveauregelung
- 9: Sterilisationsfilter
- 91: Sterilisationsfilter
- 92: Sterilisationsfilter
- 93: Sterilisationsfilter
- 94: Sterilisationsfilter
- 10: Kondensat

## Patentansprüche

1. Verfahren zur Dampfsterilisation in einem Autoklaven (1), wobei das Verfahren umfasst:
- Erzeugung von gasförmigem Wasser in einem Sofortdampferzeuger (3),
- Bereitstellung des gasförmigen Wassers in einer Kammer (2) des Autoklaven (1) zur Sterilisation von in der Kammer (2) angeordnetem Sterilgut,
- Einstellung einer Sterilisationsbedingung in der Kammer (2) für die Dauer einer Haltezeit,
- **dadurch gekennzeichnet, dass** sämtliches während der Haltezeit in der Kammer (2) befindliches Wasser sterilisiert wird.

2. Verfahren nach Anspruch 1, **dadurch gekennzeichnet, dass** sämtliches während der Haltezeit in der Kammer (2) befindliches Wasser vor der Bereitstellung in der Kammer (2) sterilisiert wird oder nach der Bereitstellung in der Kammer (2) sterilisiert wird oder vor der Bereitstellung in der Kammer (2) teilsterilisiert und nach der Bereitstellung in der Kammer (2) vollständig sterilisiert wird.

3. Verfahren nach einem der vorhergehenden Ansprüche, **dadurch gekennzeichnet, dass** eine Einstellung der Sterilisationsbedingung während der Haltezeit ausschließlich durch Verdampfung von in der Kammer (2) anfallendem Kondensat (10) erfolgt oder dass vor Beginn der Haltezeit Wasser in die Kammer (2) gespeist wird und eine Einstellung der Sterilisationsbedingung während der Haltezeit ausschließlich durch Verdampfung des vor der Haltezeit in die Kammer (2) gespeisten Wassers und durch Verdampfung von in der Kammer (2) anfallendem Kondensat (10) erfolgt.

4. Verfahren nach einem der vorhergehenden Ansprüche, **dadurch gekennzeichnet, dass** ein in der Kammer (2) anfallendes Kondensat (10) oder in der Kammer (2) anfallendes Kondensat (10) und vor Beginn der Haltezeit in die Kammer (2) gespeistes Wasser in der Kammer (2) verdampft wird, wobei die Verdampfung mittels eines in der Kammer (2) angeordneten Heizelementes (21) erfolgt oder wobei die Verdampfung mittels des Sofortdampferzeugers (3) erfolgt, wobei der Sofortdampferzeuger (3) in der Kammer (2) angeordnet ist.

5. Verfahren nach einem der Ansprüche 1 bis 3, **dadurch gekennzeichnet, dass** ein in der Kammer (2) anfallendes Kondensat (10) oder in der Kammer (2) anfallendes Kondensat (10) und vor Beginn der Haltezeit in die Kammer (2) gespeistes Wasser außerhalb der Kammer (2) verdampft wird, wobei die Verdampfung mittels des Sofortdampferzeugers (3) erfolgt, wobei der Sofortdampferzeuger (3) außerhalb der Kammer (2) angeordnet ist.

6. Verfahren nach Anspruch 5, **dadurch gekennzeichnet, dass** das in der Kammer (2) anfallende Kondensat (10) oder das in der Kammer (2) anfallende Kondensat (10) und das vor Beginn der Haltezeit in die Kammer (2) gespeistes Wasser mittels einer Pumpe (31) aus der Kammer (2) in den außerhalb der Kammer (2) angeordneten Sofortdampferzeuger (3) gepumpt wird.

7. Verfahren nach Anspruch 1 oder 2, **dadurch gekennzeichnet, dass** eine Einstellung der Sterilisationsbedingung während der Haltezeit auch durch ein Nachspeisen von gasförmigem Wasser in die Kammer (2) erfolgt.

8. Verfahren nach Anspruch 7, **dadurch gekennzeichnet, dass** der Sofortdampferzeuger (3) ein mittels eines Ventils (82) gegenüber der Kammer (2) abgeriegeltes Dampfreservoir (8) aufweist und im Sofortdampferzeuger (3) gasförmiges Wasser auf Vorrat erzeugt wird und während der Haltezeit zur Einstellung der Sterilisationsbedingung in die Kammer (2) geleitet wird.

9. Verfahren nach Anspruch 7, **dadurch gekennzeichnet, dass** das Wasser vor der Zufuhr zum Sofortdampferzeuger (3) sterilisiert wird.

10. Verfahren nach Anspruch 9, **dadurch gekennzeichnet, dass** die Sterilisation des Wassers in einem dem Sofortdampferzeuger (3) vorgelagerten Vorlagebehälter (6) durch Erhitzen auf eine Temperatur oberhalb einer Sterilisationstemperatur erfolgt oder dass die Sterilisation des Wassers in einer in oder an der Kammer (2) angeordneten Spirale (7) erfolgt, wobei die Spirale (7) ein für einen Sterilisationsvorgang benötigtes Wasservolumen während der Haltezeit speichert; oder, dass das Wasser vor der Zufuhr zum Sofortdampferzeuger (3) mittels eines Sterilfilters (9, 91, 92) sterilisiert wird und/oder das im Sofortdampferzeuger (3) erzeugte gasförmige Wasser mittels eines Sterilfilters (9, 93, 94) vor der Bereitstellung in der Kammer (2) sterilisiert wird.

11. Autoklav (1) zur Sterilisation von Sterilgut, wobei der Autoklav (1) aufweist:
- einen Sofortdampferzeuger (3) zur Erzeugung von gasförmigem Wasser;
- eine Regelungseinheit zur Einstellung einer Sterilisationsbedingung zur Sterilisation von Sterilgut in einer Kammer (2) des Autoklaven (1) für die Dauer einer Haltezeit;
- **dadurch gekennzeichnet, dass** der Autoklav (1) ausgeführt ist, sämtliches während der Haltezeit in der Kammer (2) befindliches Wasser zu sterilisieren, so dass am Ende der Haltezeit in der Kammer (2) ausschließlich steriles Wasser vorliegt.

12. Autoklav (1) nach Anspruch 11, **dadurch gekennzeichnet, dass** der Autoklav (1) ausgeführt ist, sämtliches während der Haltezeit in der Kammer (2) befindliches Wasser vor der Bereitstellung in der Kammer (2) zu sterilisieren oder nach der Bereitstellung in der Kammer (2) zu sterilisieren oder vor der Bereitstellung in der Kammer (2) zu teilsterilisieren und nach der Bereitstellung in der Kammer (2) vollständig zu sterilisieren.

13. Autoklav (1) nach Anspruch 11 oder 12, **dadurch gekennzeichnet, dass** der Sofortdampferzeuger (3) außerhalb der Kammer (2) angeordnet ist und der Autoklav (1) einen dem Sofortdampferzeuger (3) vorgelagerten beheizten Vorlagebehälter (8) zur Erhitzung von Wasser auf eine Temperatur oberhalb einer Sterilisationstemperatur aufweist, oder der Autoklav (1) eine dem Sofortdampferzeuger (3) vorgelagerte, in der Kammer (2) angeordnete Spirale (7) zur Speicherung eines für einen Sterilisationsvorgang benötigten Wasservolumens aufweist, oder der Autoklav (1) ein mittels eines Ventils (82) gegenüber der Kammer (2) abgeriegeltes Dampfreservoir (8) des Sofortdampferzeugers (3) aufweist, oder der Autoklav (1) ein oder mehrere dem Sofortdampferzeuger (3) vorgelagerte und/oder nachgelagerte Sterilfilter (9, 91, 92, 93, 94) aufweist.

14. Autoklav (1) nach Anspruch 11 oder 12, **dadurch gekennzeichnet, dass** der Autoklav (1) ausgeführt ist, eine Einstellung der Sterilisationsbedingung in der Kammer (2) für die Dauer der Haltezeit ausschließlich mittels Verdampfung des in der Kammer (2) anfallenden Kondensats (10) vorzunehmen oder der Autoklav (1) ausgeführt ist, flüssiges Wasser vor Beginn der Haltezeit in die Kammer (2) zu speisen und eine Einstellung der Sterilisationsbedingung in der Kammer (2) für die Dauer der Haltezeit ausschließlich mittels Verdampfung des vor Beginn der Haltezeit in die Kammer (2) gespeisten Wassers und mittels Verdampfung des in der Kammer (2) anfallenden Kondensats (10) vorzunehmen.

15. Autoklav (1) nach Anspruch 14, **dadurch gekennzeichnet, dass** der Autoklav (1) zur Verdampfung des in der Kammer (2) anfallenden Kondensats (10) oder zur Verdampfung des in der Kammer (2) anfallendes Kondensat (10) und des vor Beginn der Haltezeit in die Kammer (2) gespeistes Wasser ein in der Kammer (2) angeordnetes Heizelement (21) aufweist, oder dass der Autoklav (1) zur Verdampfung des in der Kammer (2) anfallenden Kondensats (10) oder zur Verdampfung des in der Kammer (2) anfallendes Kondensat (10) und des vor Beginn der Haltezeit in die Kammer (2) gespeistes Wasser den Sofortdampferzeuger (3) sowie eine Pumpe (31) zur Zufuhr von Kondensat (10) aus der Kammer (2) in den Sofortdampferzeuger (3) aufweist, wobei der Sofortdampferzeuger (3) außerhalb der Kammer (2) angeordnet ist, oder dass der Autoklav (1) zur Verdampfung des in der Kammer (2) anfallenden Kondensats (10) oder zur Verdampfung des in der Kammer (2) anfallendes Kondensat (10) und des vor Beginn der Haltezeit in die Kammer (2) gespeistes Wasser den Sofortdampferzeuger (3) aufweist, wobei der Sofortdampferzeuger (3) in der Kammer (2) angeordnet ist.

## Geänderte Patentansprüche

### Geänderte Patentansprüche gemäss Regel 137(2) EPÜ.

1. Verfahren zur Dampfsterilisation in einem Autoklaven (1), wobei das Verfahren umfasst:
- Erzeugung von gasförmigem Wasser in einem Sofortdampferzeuger (3),
- Bereitstellung des gasförmigen Wassers in einer Kammer (2) des Autoklaven (1) zur Sterilisation von in der Kammer (2) angeordnetem Sterilgut,
- Einstellung einer Sterilisationsbedingung in der Kammer (2) für die Dauer einer Haltezeit,
- **dadurch gekennzeichnet, dass** sämtliches während der Haltezeit in der Kammer (2) befindliches Wasser sterilisiert wird.

2. Verfahren nach Anspruch 1, **dadurch gekennzeichnet, dass** sämtliches während der Haltezeit in der Kammer (2) befindliches Wasser vor der Bereitstellung in der Kammer (2) sterilisiert wird oder nach der Bereitstellung in der Kammer (2) sterilisiert wird oder vor der Bereitstellung in der Kammer (2) teilsterilisiert und nach der Bereitstellung in der Kammer (2) vollständig sterilisiert wird.

3. Verfahren nach einem der vorhergehenden Ansprüche, **dadurch gekennzeichnet, dass** eine Einstellung der Sterilisationsbedingung während der Haltezeit ausschließlich durch Verdampfung von in der Kammer (2) anfallendem Kondensat (10) erfolgt oder dass vor Beginn der Haltezeit Wasser in die Kammer (2) gespeist wird und eine Einstellung der Sterilisationsbedingung während der Haltezeit ausschließlich durch Verdampfung des vor der Haltezeit in die Kammer (2) gespeisten Wassers und durch Verdampfung von in der Kammer (2) anfallendem Kondensat (10) erfolgt.

4. Verfahren nach einem der vorhergehenden Ansprüche, **dadurch gekennzeichnet, dass** ein in der Kammer (2) anfallendes Kondensat (10) oder in der Kammer (2) anfallendes Kondensat (10) und vor Beginn der Haltezeit in die Kammer (2) gespeistes Wasser in der Kammer (2) verdampft wird, wobei die Verdampfung mittels eines in der Kammer (2) angeordneten Heizelementes (21) erfolgt oder wobei die Verdampfung mittels des Sofortdampferzeugers (3) erfolgt, wobei der Sofortdampferzeuger (3) in der Kammer (2) angeordnet ist.

5. Verfahren nach einem der Ansprüche 1 bis 3, **dadurch gekennzeichnet, dass** ein in der Kammer (2) anfallendes Kondensat (10) oder in der Kammer (2) anfallendes Kondensat (10) und vor Beginn der Haltezeit in die Kammer (2) gespeistes Wasser außerhalb der Kammer (2) verdampft wird, wobei die Verdampfung mittels des Sofortdampferzeugers (3) erfolgt, wobei der Sofortdampferzeuger (3) außerhalb der Kammer (2) angeordnet ist.

6. Verfahren nach Anspruch 5, **dadurch gekennzeichnet, dass** das in der Kammer (2) anfallende Kondensat (10) oder das in der Kammer (2) anfallende Kondensat (10) und das vor Beginn der Haltezeit in die Kammer (2) gespeistes Wasser mittels einer Pumpe (31) aus der Kammer (2) in den außerhalb der Kammer (2) angeordneten Sofortdampferzeuger (3) gepumpt wird.

7. Verfahren nach Anspruch 1 oder 2, **dadurch gekennzeichnet, dass** eine Einstellung der Sterilisationsbedingung während der Haltezeit auch durch ein Nachspeisen von gasförmigem Wasser in die Kammer (2) erfolgt, wobei der Sofortdampferzeuger (3) ein mittels eines Ventils (82) gegenüber der Kammer (2) abgeriegeltes Dampfreservoir (8) aufweist und im Sofortdampferzeuger (3) gasförmiges Wasser auf Vorrat erzeugt wird und während der Haltezeit zur Einstellung der Sterilisationsbedingung in die Kammer (2) geleitet wird.

8. Verfahren nach Anspruch 1 oder 2, **dadurch gekennzeichnet, dass** eine Einstellung der Sterilisationsbedingung während der Haltezeit auch durch ein Nachspeisen von gasförmigem Wasser in die Kammer (2) erfolgt, wobei das Wasser vor der Zufuhr zum Sofortdampferzeuger (3) sterilisiert wird.

9. Verfahren nach Anspruch 8, **dadurch gekennzeichnet, dass** die Sterilisation des Wassers in einem dem Sofortdampferzeuger (3) vorgelagerten Vorlagebehälter (6) durch Erhitzen auf eine Temperatur oberhalb einer Sterilisationstemperatur erfolgt oder dass die Sterilisation des Wassers in einer in oder an der Kammer (2) angeordneten Spirale (7) erfolgt, wobei die Spirale (7) ein für einen Sterilisationsvorgang benötigtes Wasservolumen während der Haltezeit speichert; oder, dass das Wasser vor der Zufuhr zum Sofortdampferzeuger (3) mittels eines Sterilfilters (9, 91, 92) sterilisiert wird und/oder das im Sofortdampferzeuger (3) erzeugte gasförmige Wasser mittels eines Sterilfilters (9, 93, 94) vor der Bereitstellung in der Kammer (2) sterilisiert wird.

10. Autoklav (1) zur Sterilisation von Sterilgut, wobei der Autoklav (1) aufweist:
- einen Sofortdampferzeuger (3) zur Erzeugung von gasförmigem Wasser;
- eine Regelungseinheit zur Einstellung einer Sterilisationsbedingung zur Sterilisation von Sterilgut in einer Kammer (2) des Autoklaven (1) für die Dauer einer Haltezeit;
- **dadurch gekennzeichnet, dass** der Autoklav (1) ausgeführt ist, sämtliches während der Haltezeit in der Kammer (2) befindliches Wasser zu sterilisieren, so dass am Ende der Haltezeit in der Kammer (2) ausschließlich steriles Wasser vorliegt.

11. Autoklav (1) nach Anspruch 10, **dadurch gekennzeichnet, dass** der Autoklav (1) ausgeführt ist, sämtliches während der Haltezeit in der Kammer (2) befindliches Wasser vor der Bereitstellung in der Kammer (2) zu sterilisieren oder nach der Bereitstellung in der Kammer (2) zu sterilisieren oder vor der Bereitstellung in der Kammer (2) zu teilsterilisieren und nach der Bereitstellung in der Kammer (2) vollständig zu sterilisieren.

12. Autoklav (1) nach Anspruch 10 oder 11, **dadurch gekennzeichnet, dass** der Sofortdampferzeuger (3) außerhalb der Kammer (2) angeordnet ist und der Autoklav (1) einen dem Sofortdampferzeuger (3) vorgelagerten beheizten Vorlagebehälter (8) zur Erhitzung von Wasser auf eine Temperatur oberhalb einer Sterilisationstemperatur aufweist, oder der Autoklav (1) eine dem Sofortdampferzeuger (3) vorgelagerte, in der Kammer (2) angeordnete Spirale (7) zur Speicherung eines für einen Sterilisationsvorgang benötigten Wasservolumens aufweist, oder der Autoklav (1) ein mittels eines Ventils (82) gegenüber der Kammer (2) abgeriegeltes Dampfreservoir (8) des Sofortdampferzeugers (3) aufweist, oder der Autoklav (1) ein oder mehrere dem Sofortdampferzeuger (3) vorgelagerte und/oder nachgelagerte Sterilfilter (9, 91, 92, 93, 94) aufweist.

13. Autoklav (1) nach Anspruch 10 oder 11, **dadurch gekennzeichnet, dass** der Autoklav (1) ausgeführt ist, eine Einstellung der Sterilisationsbedingung in der Kammer (2) für die Dauer der Haltezeit ausschließlich mittels Verdampfung des in der Kammer (2) anfallenden Kondensats (10) vorzunehmen oder der Autoklav (1) ausgeführt ist, flüssiges Wasser vor Beginn der Haltezeit in die Kammer (2) zu speisen und eine Einstellung der Sterilisationsbedingung in der Kammer (2) für die Dauer der Haltezeit ausschließlich mittels Verdampfung des vor Beginn der Haltezeit in die Kammer (2) gespeisten Wassers und mittels Verdampfung des in der Kammer (2) anfallenden Kondensats (10) vorzunehmen.

14. Autoklav (1) nach Anspruch 13, **dadurch gekennzeichnet, dass** der Autoklav (1) zur Verdampfung des in der Kammer (2) anfallenden Kondensats (10) oder zur Verdampfung des in der Kammer (2) anfallendes Kondensat (10) und des vor Beginn der Haltezeit in die Kammer (2) gespeistes Wasser ein in der Kammer (2) angeordnetes Heizelement (21) aufweist, oder dass der Autoklav (1) zur Verdampfung des in der Kammer (2) anfallenden Kondensats (10) oder zur Verdampfung des in der Kammer (2) anfallendes Kondensat (10) und des vor Beginn der Haltezeit in die Kammer (2) gespeistes Wasser den Sofortdampferzeuger (3) sowie eine Pumpe (31) zur Zufuhr von Kondensat (10) aus der Kammer (2) in den Sofortdampferzeuger (3) aufweist, wobei der Sofortdampferzeuger (3) außerhalb der Kammer (2) angeordnet ist, oder dass der Autoklav (1) zur Verdampfung des in der Kammer (2) anfallenden Kondensats (10) oder zur Verdampfung des in der Kammer (2) anfallendes Kondensat (10) und des vor Beginn der Haltezeit in die Kammer (2) gespeistes Wasser den Sofortdampferzeuger (3) aufweist, wobei der Sofortdampferzeuger (3) in der Kammer (2) angeordnet ist.
